(19) Europäisches Patentamt
European Patent Office
Office européen des brevets

(11) EP 4 741 374 A1

(12) EUROPEAN PATENT APPLICATION
published in accordance with Art. 153(4) EPC

(43) Date of publication:
**13.05.2026 Bulletin 2026/20**

(21) Application number: **24835227.0**

(22) Date of filing: **19.06.2024**

(51) International Patent Classification (IPC):
***C07C 405/00*** (2006.01) ***A61K 31/335*** (2006.01)
***A61K 31/222*** (2006.01) ***A61P 9/00*** (2006.01)

(52) Cooperative Patent Classification (CPC):
**C07C 69/712; A61K 31/222; A61K 31/335; A61K 31/5575; A61P 9/00; A61P 11/00; C07C 219/06;** C07C 2603/14

(86) International application number:
**PCT/CN2024/100071**

(87) International publication number:
**WO 2025/007744 (09.01.2025 Gazette 2025/02)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **03.07.2023 CN 202310804817**

(71) Applicants:
- **CF PharmTech HongKong Limited
Hong Kong 999077 (HK)**
- **CF Pharmtech Guangzhou Limited
Guangzhou, Guangdong 510700 (CN)**
- **CF PharmTech USA, Inc.
Lewes, County of Sussex, Delaware 19958 (US)**
- **CF Pharmtech, Inc.
Jiangsu 215143 (CN)**

(72) Inventors:
- **XU, Lin
Suzhou, Jiangsu 215000 (CN)**
- **QUAN, Mengxue
Suzhou, Jiangsu 215000 (CN)**
- **CHE, Lin
Suzhou, Jiangsu 215000 (CN)**
- **ZHANG, Liang
Suzhou, Jiangsu 215000 (CN)**
- **LI, Xiujuan
Suzhou, Jiangsu 215000 (CN)**
- **XU, Beibei
Suzhou, Jiangsu 215000 (CN)**
- **DONG, Jingwei
Suzhou, Jiangsu 215000 (CN)**
- **LIU, Xiao
Suzhou, Jiangsu 215000 (CN)**
- **TAO, Hongfu
Suzhou, Jiangsu 215000 (CN)**
- **LIANG, Bill Wenqing
Suzhou, Jiangsu 215000 (CN)**

(74) Representative: **Forresters IP LLP
Skygarden
Erika-Mann-Straße 11
80636 München (DE)**

# (54) TREPROSTINIL DERIVATIVE, AND PREPARATION METHOD THEREFOR AND USE THEREOF

(57) The present invention relates to the field of chemistry and medicine. Provided is a treprostinil derivative represented by formula I, or an isomer or a pharmaceutically acceptable salt thereof, wherein each group is as defined in the description. The compound of formula I, or the isomer or the pharmaceutically acceptable salt thereof can remain in the lung for a long time and slowly release treprostinil at an effective concentration after pulmonary administration, and can therefore be used for treating pulmonary hypertension and pulmonary fibrosis. The pulmonary administration of the compound of formula I, or the isomer or the pharmaceutically acceptable salt thereof provided in the present invention can greatly reduce the administration dosage, provide a lower administration frequency and a broader therapeutic window, and reduce systemic side effects and local lung irritation. Therefore, the drug safety and drug tolerance of a patient are greatly improved, and a new treatment choice is provided for a patient with pulmonary hypertension and pulmonary fibrosis.

EP 4 741 374 A1

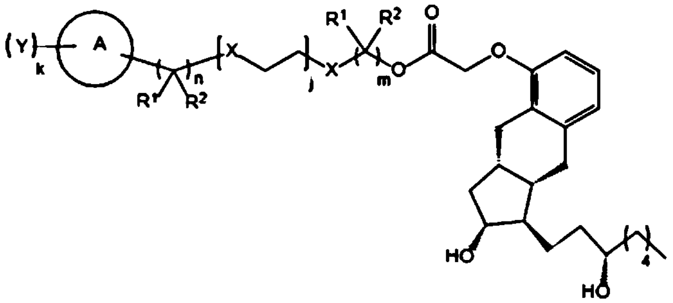

Formula I

## Description

TECHNICAL FIELD

The present invention relates to the field of chemical medicine, and particularly to a treprostinil derivative as shown in Formula I, a preparation method therefor and a use thereof.

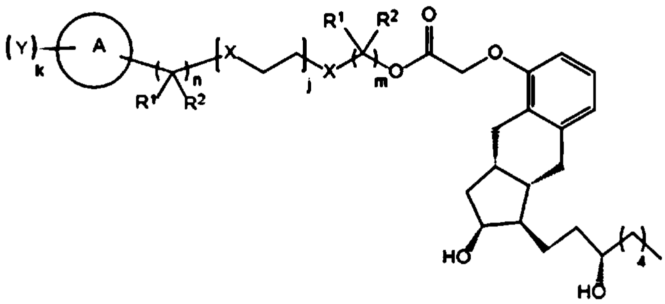

Formula I

BACKGROUND

Pulmonary hypertension (PH) is a clinical and pathophysiological syndrome defined by a mean pulmonary arterial pressure ≥25 mmHg at rest, as measured by right heart catheterization at sea level. The disease is characterized by pulmonary vascular disease and remodeling, arterial luminal narrowing and impaired vasodilation. These pathological changes lead to increased pulmonary arterial pressure (PAP) and pulmonary vascular resistance (PVR), which in turn limits the capacity of the right ventricle to pump blood to lungs through pulmonary arteries, resulting in shortness of breath and eventually leading to right heart failure and death. PH has diverse etiologies and complex pathogenesis. It is clinically classified into 5 groups: (1) Pulmonary arterial hypertension (PAH); (2) PH due to left heart diseases (PH-LHD); (3) PH due to lung diseases and/or hypoxia; (4) Chronic thromboembolic pulmonary hypertension (CTEPH) and (or) PH due to pulmonary arterial obstructive diseases; and (5) PH with unclear and (or) multifactorial mechanism.

According to statistics, the prevalence rate of the PH is approximately 1% in general population, but increased to 10% in people over 65 years old. Among various types of PH, PH due to left heart diseases and PH due to lung diseases and/or hypoxia are the most common types in clinical practice. For PH due to lung diseases and/or hypoxia, chronic obstructive pulmonary disease (COPD), interstitial lung disease, and pulmonary fibrosis are the most common causes. The causes of idiopathic pulmonary arterial hypertension (IPAH) are currently unknown. Although the incidence of PAH is low, it is often referred to as "cancer of the cardiovascular system". Epidemiological data indicate that the 5-year survival rate of patients with idiopathic and heritable PAH is only 20.8% following conventional treatment. Furthermore, due to a lack of professional disease management capacity and effective patient-physician communication mode, disease remains suboptimal in most PAH patients even after discharge.

At present, the treatment of PAH still primarily targets three signaling pathways: prostacyclin (PGI2), endothelin-1 (ET-1) and nitric oxide (NO). Among these, the PGI2 signaling pathway is the most important and represents the only class of targeted drugs among the three that can reduce mortality in patients with PAH. The PGI2 is a potent vasodilator, which binds to prostaglandin receptors (IP receptor,EP receptor), activates adenylate cyclase, and increases the intracellular concentration of cAMP, thereby inducing vasodilation. In addition, PGI2 also has antiplatelet, antiproliferative, and anti-inflammatory effects. In patients with PAH, the synthesis of PGI2 and the expression of the IP receptor are decreased. PGI2 analogues function by mimicking the binding of PGI2 to the IP receptor.

Currently marketed prostacyclin drugs, which primarily include epoprostenol, iloprost, beraprost and treprostinil, are mostly expensive and plagued by problems such as a short half-life, poor chemical stability, and significant side effects, and often need to be administered by continuous intravenous or subcutaneous infusion, which can lead to severe injection site pain and infection. Moreover, if the intravenous infusion is abruptly interrupted, patients may also face the risk of fatal rebound pulmonary hypertension. Although an inhaled form of treprostinil (trade name: Tyvaso) has been approved for the treatment of the PAH and its efficacy has been established in clinical studies for patients with idiopathic pulmonary interstitial pneumonia (IIP), including idiopathic pulmonary fibrosis (IPF) and combined pulmonary fibrosis and emphysema (CPFE), the short half-life (about 30 minutes) necessitates frequent administration of inhaled treprostinil to maintain pulmonary vasodilation. In addition, the inhalation of the treprostinil is associated with local and systemic adverse events, including cough, headache, throat irritation, and the like, thereby affecting the tolerance and the curative effect, and complicating the clinical application.

To overcome these shortcomings, researchers have evaluated numerous alternative therapeutic strategies, including liposomal encapsulation, novel drug delivery devices, particle engineering using modern technologies, and

prodrug modification of treprostinil. Insmed has developed treprostinil palmitate (C16-TR), an ester prodrug of treprostinil that undergoes slow hydrolysis to provide sustained release of treprostinil over an extended period. The structural formulas of treprostinil and C16-TR are shown below.

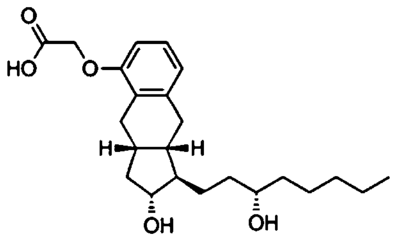

Treprostinil

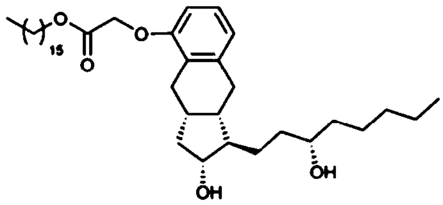

Treprostinil palmitate (C16-TR)

C16-TR, a sustained-release prodrug designed for inhalation administration, aims to improve patient compliance and reduce side effects of systemic administration. Clinical studies show that the C16-TR has good therapeutic efficacy. At a low dose (54 μg/kg), subjects experienced no side effects such as cough or sore throat. However, as the dose increased (170 μg/kg and 340 μg/kg), subjects developed cough and sore throat. Due to the intrinsic physicochemical properties of the C16-TR compound, it cannot be directly used as a solution for nebulization solution. Initial efforts therefore focused on developing solid lipid nanoparticles (LNP) and utilizing PEG-modified lipids to enhance the stability of the drug and particles, followed by formulating the LNPs as a suspension for delivery to the lungs. However, this formulation technology is complex and expensive, with high industrial costs, potentially resulting in high treatment expenses for treatment. Currently, no commercial inhalation product utilizing LNP technology exists, and the long-term safety of its complex excipient components in the lungs has not been certified by regulatory agencies. During aerosol development, due to a limited solubility of the C16-TR in a HFA propellant containing ethanol, surfactants such as DSPE-PEG must be added for solubilization. Long-term exposure to a high-dose PEGylated lipid may cause potential pulmonary toxicity. Furthermore, since PEGylated lipids possess certain immunogenicity, their long-term use may also induce adverse effects such as immune responses in the body. It has also been reported that the C16-TR in the dissolved state can react with ethanol, leading to substitution of the C16 ester by an ethyl ester. Therefore, the C16-TR faces significant challenges in formulation development.

In summary, the exploration of more suitable compounds or dosage forms holds significant and farreaching clinical value. This endeavor aims to further improve the in vivo metabolic characteristics of treprostinil to reduce its administration dosage and frequency, lower its toxic side effects, enhance the physicochemical properties of the compound, decrease industrial preparation costs, and ultimately provide patients with a safer, more convenient and more economical regimen, thereby improving their quality of life and prolonging their survial, and having a very positive and far-reaching clinical value.

SUMMARY

Aiming at the problems in the prior art, the present invention provides a treprostinil derivative and a medical use thereof.

In one aspect, the present invention provides a compound of Formula I, an isomer or a pharmaceutically acceptable salt thereof,

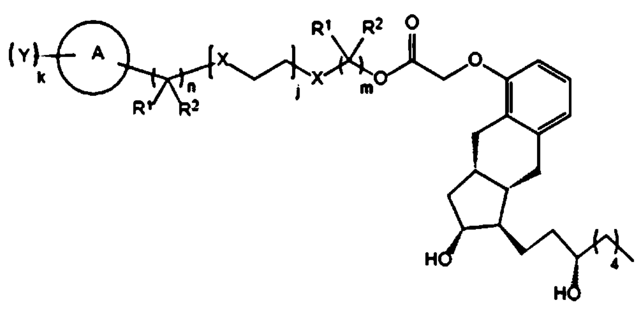

Formula I

wherein,

X is each independently selected from the group consisting of O and S;

A is a cyclic group, and the cyclic group is selected from the group consisting of phenyl and 5- to 10-membered heteroaryl, wherein the heteroatom in the 5- to 10-membered heteroaryl is selected from one or more of N, O and S;

$R^1$ and $R^2$ are each independently selected from the group consisting of H, $C_1$-$C_6$ alkyl, $C_2$-$C_6$ alkenyl, $C_2$-$C_6$ alkynyl, $C_1$-$C_6$ alkoxy, halogen atom, hydroxyl, amino, cyano, nitro and -$ONO_2$, or $R^1$ and $R^2$ form a cyclic group together with the carbon atom to which $R^1$ and $R^2$ are attached, wherein, the $C_1$-$C_6$ alkyl, $C_2$-$C_6$ alkenyl and $C_1$-$C_6$ alkoxy are optionally substituted by one or more substituents selected from the following groups: cycloalkyl, aryl, heteroaryl, -$NR^3R^4$, halogen atom, hydroxyl, cyano, mercapto, nitro or -$ONO_2$;

$R^3$ and $R^4$ are each independently selected from the group consisting of H, $C_1$-$C_6$ alkyl and $C_3$-$C_6$ cycloalkyl, or $R^3$ and $R^4$ form a 5-8 membered heterocyclic ring together with the nitrogen atom to which $R^3$ and $R^4$ are attached;

Y is each independently selected from the group consisting of $C_1$-$C_6$ alkyl, $C_2$-$C_6$ alkenyl, $C_2$-$C_6$ alkynyl, halogen, hydroxyl, carboxyl, cyano, mercapto, $C_1$-$C_6$ alkoxy, $C_1$-$C_6$ alkylamino and 3- to 8- membered cycloalkyl;

m is an integer from 4 to 16;

n is an integer from 0 to 8;

j is an integer from 0 to 2; and

k is an integer from 0 to 3.

In some embodiments, A is phenyl; or A is 5-8 membered heteroaryl (for example, pyridyl), wherein the heteroatom in 5-8 membered heteroaryl is N.

In some embodiments, X is each independently O.

According to the compound of Formula I, the isomer or the pharmaceutically acceptable salt thereof in the present invention, $R^1$ and $R^2$ are each independently selected from the H or $C_{1-6}$ alkyl, wherein the $C_{1-6}$ alkyl is optionally substituted by one or more substituents selected from the following groups: halogen atom, hydroxyl, cyano, amino, nitro, -$ONO_2$ and mercapto.

According to the compound of Formula I, the isomer or the pharmaceutically acceptable salt thereof in the present invention, Y is each independently selected from the group consisting of $C_1$-$C_6$ alkyl, halogen, cyano, and $C_1$-$C_6$ alkoxy, preferably halogen and $C_1$-$C_6$ alkyl, and more preferably chlorine.

According to the compound of Formula I, the isomer or the pharmaceutically acceptable salt thereof in the present invention, Y is an ortho or para substituent.

According to the compound of Formula I, the isomer or the pharmaceutically acceptable salt thereof in the present invention, m is an integer from 6 to 10;

According to the compound of Formula I, the isomer or the pharmaceutically acceptable salt thereof in the present invention, the n is an integer from 2 to 8, preferably from 4 to 6.

According to the compound of Formula I, the isomer or the pharmaceutically acceptable salt thereof in the present invention, the j is an integer from 0 to 1.

According to the compound of Formula I, the isomer or the pharmaceutically acceptable salt thereof in the present invention, the k is an integer from 0 to 2.

According to the compound of Formula I, the isomer or the pharmaceutically acceptable salt thereof in the present invention, the compound is selected from:

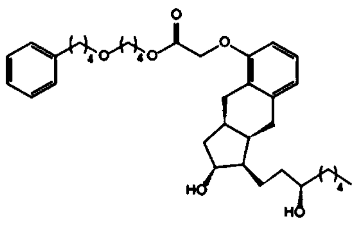
,
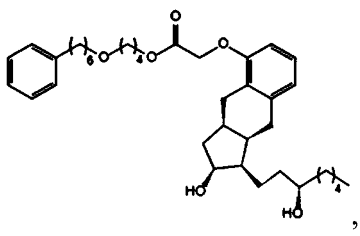
,
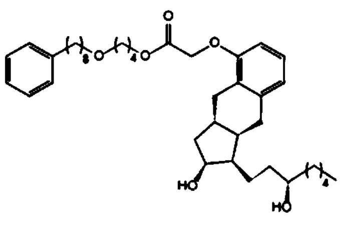
,

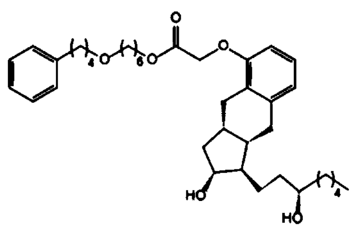
,
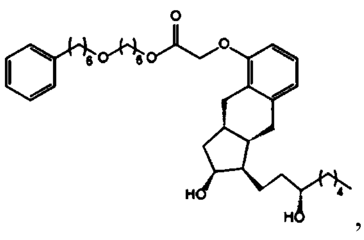
,
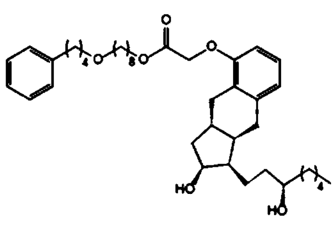
,

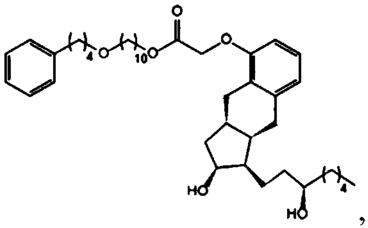,
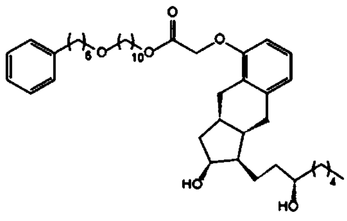,
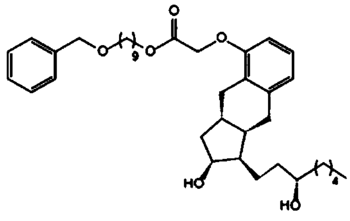,
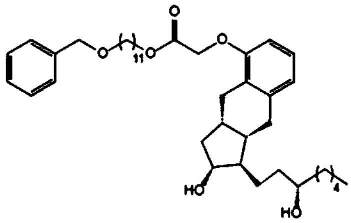,
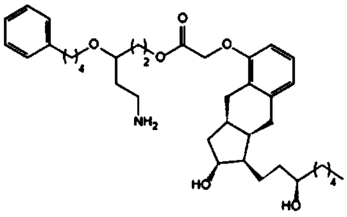,
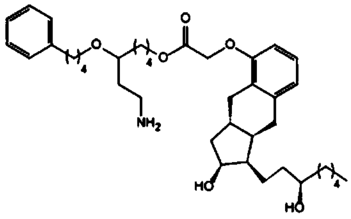,
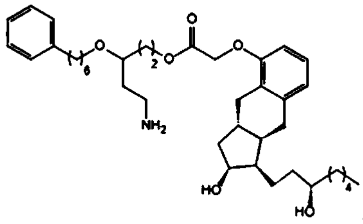,
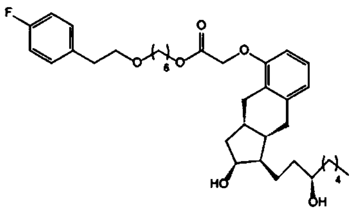,
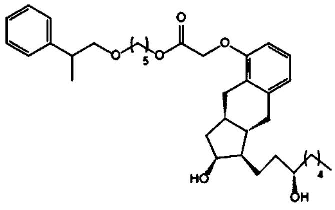,
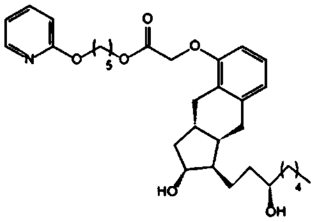,
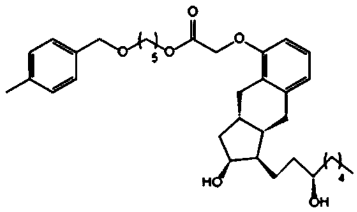,
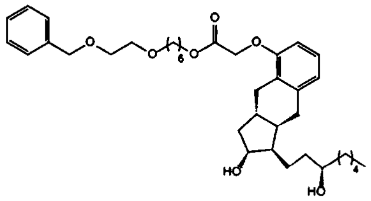,

and

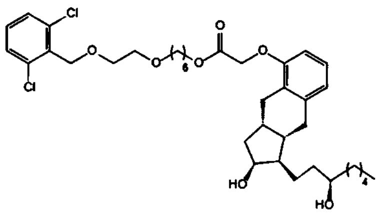.

The compound of Formula I, the isomer or the pharmaceutically acceptable salt thereof in the present invention is sustainably and slowly converted into treprostinil, and has good solubility and stability in an alcohol solvent, especially when X is O, j is 0 and A is phenyl.

In a second aspect, the present invention provides an isotope substitute of the compound of Formula I, the isomer or the pharmaceutically acceptable salt thereof above, wherein the isotope substitute is preferably a deuterium atom substitute.

According to the compound of Formula I, the isomer or the pharmaceutically acceptable salt or the isotope substitute thereof provided by the present invention, due to a unique ester side chain structure, the compound exhibit unique microkinetic characteristics of diffusion in lungs after pulmonary administration, and the lipophilic side chain structure is highly distributed in a phospholipid layer of a cell membrane surface, so that the compound of Formula I is slowly converted and releases treprostinil in a local microenvironment around a receptor, and the released treprostinil can be diffused to a prostaglandin receptor binding site on the cell surface more effectively, thereby exerting an effective and lasting drug action.

In a third aspect, the present invention provides a pharmaceutical composition, which comprises at least one compound of Formula I, the isomer or the pharmaceutically acceptable salt, or the isotope substitute thereof above, and a pharmaceutically acceptable excipient, wherein the pharmaceutically acceptable excipient comprises a carrier, a diluent, an excipient, and the like.

According to the pharmaceutical composition in the present invention, a unit dose of the pharmaceutical composition is 0.001 mg-1000 mg, preferably 0.001 mg-500 mg, preferably 0.001 mg-100 mg, and more preferably 0.001 mg-10 mg.

According to the pharmaceutical composition in the present invention, based on a total weight of the composition, the pharmaceutical composition contains 0.01%-99.99% of the compound above. According to the pharmaceutical composition in the present invention, the pharmaceutical composition contains 0.01%-90% of the compound above. According to the pharmaceutical composition in the present invention, the pharmaceutical composition contains 0.01%-80% of the compound above. According to the pharmaceutical composition in the present invention, the pharmaceutical composition contains 0.01%-70% of the compound above. According to the pharmaceutical composition in the present invention, the pharmaceutical composition contains 0.01%-60% of the compound above. According to the pharmaceutical composition in the present invention, the pharmaceutical composition contains 0.01%-50% of the compound above. The remaining weight in the composition is contributed by the pharmaceutically acceptable carrier, diluent or excipient.

According to the pharmaceutical composition in the present invention, the pharmaceutical composition may further contain other active agents besides the compound of the present invention.

In a fourth aspect, the present invention provides a use of the compound of Formula I, the isomer or the pharmaceutically acceptable salt, the isotope substitute or the pharmaceutical composition thereof in the present invention, in the preparation of a medicament for treating pulmonary hypertension (PH).

In a fifth aspect, the present invention provides a use of the compound of Formula I, the isomer or the pharmaceutically acceptable salt thereof , the isotope substitute, or the pharmaceutical composition thereof in the present invention in the preparation of a medicament for treating pulmonary fibrosis.

According to the use in the present invention, the use comprises administering an effective dose of the compound of Formula I, the isomer or the pharmaceutically acceptable salt, the isotope substitute, or the pharmaceutical composition thereof above to a patient.

According to the use in the present invention, the administration refers to subcutaneous, oral, nasal, intravenous or pulmonary administration, preferably the pulmonary administration.

According to the use in the present invention, the pulmonary administration comprises administrating through a metered dose inhaler, a dry powder inhaler, a nebulizer and a soft mist inhaler.

According to the use in the present invention, the pulmonary administration comprises administrating through a metered dose inhaler.

According to the use in the present invention, the pulmonary administration comprises administrating through a dry powder inhaler.

According to the use in the present invention, the pulmonary administration comprises administrating through a nebulizer.

According to the use in the present invention, the pulmonary administration comprises administrating through a soft mist inhaler.

According to the use in the present invention, a patient suffering from the pulmonary hypertension is a patient with World Health Organization (WHO) group I PH, or a patient with WHO group II PH, or a patient with WHO group III PH, or a patient with WHO group IV PH, or a patient with WHO group V PH.

According to the use in the present invention, the pulmonary hypertension is selected from the group consisting of pulmonary arterial hypertension (PAH), pulmonary hypertension due to left heart diseases, pulmonary hypertension due to lung diseases and/or hypoxia, chronic thromboembolic pulmonary hypertension (CTEPH), pulmonary hypertension due to pulmonary arterial obstructive diseases, and pulmonary hypertension associated with systemic or metabolic diseases.

According to the use in the present invention, the pulmonary arterial hypertension (PAH) is selected from the group consisting of idiopathic pulmonary arterial hypertension, heritable pulmonary arterial hypertension, pulmonary arterial hypertension associated with congenital heart diseases, pulmonary arterial hypertension associated with portal hypertension, and pulmonary arterial hypertension associated with a drug or a poison.

According to the use in the present invention, the pulmonary arterial hypertension (PAH) is idiopathic pulmonary arterial hypertension.

According to the use in the present invention, the pulmonary arterial hypertension (PAH) is heritable pulmonary arterial hypertension.

According to the use in the present invention, the pulmonary arterial hypertension (PAH) is pulmonary arterial hypertension associated with portal hypertension.

According to the use in the present invention, the pulmonary hypertension (PH) is the pulmonary hypertension due to left heart diseases.

According to the use in the present invention, the pulmonary hypertension (PH) is the pulmonary hypertension due to lung diseases and/or hypoxia. The pulmonary hypertension due to lung diseases and/or hypoxia is pulmonary hypertension associated with interstitial lung diseases (ILD-PH), pulmonary hypertension due to chronic obstructive pulmonary diseases, and pulmonary hypertension due to combined pulmonary fibrosis and emphysema.

According to the use in the present invention, the pulmonary hypertension (PH) is the chronic thromboembolic pulmonary hypertension.

According to the use in the present invention, the pulmonary hypertension (PH) is the pulmonary hypertension associated with systemic or metabolic diseases.

According to the use in the present invention, the pulmonary hypertension associated with systemic or metabolic diseases is pulmonary hypertension associated with sarcoidosis.

According to the use in the present invention, the pulmonary fibrosis is selected from pulmonary interstitial fibrosis due to idiopathic interstitial pneumonias (IIPs) and pulmonary interstitial fibrosis due to connective tissue diseases.

According to the use in the present invention, the pulmonary interstitial fibrosis due to the idiopathic interstitial pneumonias (IIPs) is idiopathic fibrosis (IPF).

According to the use in the present invention, the effective dose of the compound of Formula I, the isomer or the pharmaceutically acceptable salt, the isotope substitute, or the pharmaceutical composition thereof above is administered to the patient once daily.

According to the use in the present invention, the effective dose of the compound of Formula I, the isomer or the pharmaceutically acceptable salt, the isotope substitute, or the pharmaceutical composition thereof above is administered to the patient once every two days or at a longer administration interval.

According to the use in the present invention, the effective dose of the compound of Formula I, the isomer or the pharmaceutically acceptable salt, the isotope substitute, or the pharmaceutical composition thereof above is administered to the patient twice daily.

In a sixth aspect, the present invention provides a method for treating pulmonary hypertension (PH), which comprises administering an effective dose of the compound of Formula I, the isomer or the pharmaceutically acceptable salt, the isotope substitute, or the pharmaceutical composition thereof above to a patient.

In a seventh aspect, the present invention provides a method for treating pulmonary fibrosis, which comprises administering an effective dose of the compound of Formula I, the isomer or the pharmaceutically acceptable salt, the isotope substitute, or the pharmaceutical composition thereof above to a patient.

According to the treatment method in the present invention, the administration refers to subcutaneous, oral, nasal, intravenous or pulmonary administration, preferably the pulmonary administration.

According to the treatment method in the present invention, the pulmonary administration comprises administrating through a metered dose inhaler, a dry powder inhaler, a nebulizer and a soft mist inhaler.

According to the treatment method in the present invention, the pulmonary administration comprises administrating through a metered dose inhaler.

According to the treatment method in the present invention, the pulmonary administration comprises administrating through a dry powder inhaler.

According to the treatment method in the present invention, the pulmonary administration comprises administrating through a nebulizer.

According to the treatment method in the present invention, the pulmonary administration comprises administrating through a soft mist inhaler.

According to the method for treating the pulmonary hypertension in the present invention, the patient is a patient with World Health Organization (WHO) group I PH, or a patient with WHO group II PH, or a patient with WHO group III PH, or a patient with WHO group IV PH, or a patient with WHO group V PH.

According to the method for treating the pulmonary hypertension in the present invention, the pulmonary hypertension is selected from pulmonary arterial hypertension (PAH), pulmonary hypertension due to left heart diseases, pulmonary hypertension due to lung diseases and/or hypoxia, chronic thromboembolic pulmonary hypertension (CTEPH), pulmonary hypertension due to pulmonary arterial obstructive diseases, and pulmonary hypertension associated with systemic or metabolic diseases.

According to the method for treating the pulmonary hypertension in the present invention, the pulmonary arterial hypertension (PAH) is selected from the group consisting of idiopathic pulmonary arterial hypertension, heritable pulmonary arterial hypertension, pulmonary arterial hypertension associated with congenital heart diseases, pulmonary arterial hypertension associated with portal hypertension, and pulmonary arterial hypertension associated with a drug or a poison.

According to the method for treating the pulmonary hypertension in the present invention, the pulmonary arterial hypertension (PAH) is idiopathic pulmonary arterial hypertension.

According to the method for treating the pulmonary hypertension in the present invention, the pulmonary arterial hypertension (PAH) is heritable pulmonary arterial hypertension.

According to the method for treating the pulmonary hypertension in the present invention, the pulmonary arterial hypertension (PAH) is pulmonary arterial hypertension associated with portal hypertension.

According to the method for treating the pulmonary hypertension in the present invention, the pulmonary hypertension (PH) is the pulmonary hypertension due to left heart diseases.

According to the method for treating the pulmonary hypertension in the present invention, the pulmonary hypertension (PH) is the pulmonary hypertension due to lung diseases and/or hypoxia. The pulmonary hypertension due to lung diseases and/or hypoxia is pulmonary hypertension associated with interstitial lung diseases (ILD-PH), pulmonary hypertension due to chronic obstructive pulmonary diseases, and pulmonary hypertension due to combined pulmonary fibrosis and emphysema.

According to the method for treating the pulmonary hypertension in the present invention, the pulmonary hypertension (PH) is the chronic thromboembolic pulmonary hypertension.

According to the method for treating the pulmonary hypertension in the present invention, the pulmonary hypertension (PH) is the pulmonary hypertension associated with systemic or metabolic diseases.

According to the method for treating the pulmonary hypertension in the present invention, the pulmonary hypertension associated with systemic or metabolic diseases is pulmonary hypertension associated with to sarcoidosis.

According to the method for treating the pulmonary fibrosis in the present invention, the pulmonary fibrosis is pulmonary interstitial fibrosis due to idiopathic interstitial pneumonias (IIPs) and pulmonary interstitial fibrosis due to connective tissue diseases.

According to the method for treating the pulmonary fibrosis in the present invention, the pulmonary interstitial fibrosis due to the idiopathic interstitial pneumonias (IIPs) is idiopathic fibrosis (IPF).

According to the treatment method in the present invention, the effective dose of the compound of Formula I, the isomer or the pharmaceutically acceptable salt, the isotope substitute, or the pharmaceutical composition thereof above is administered to the patient once daily.

According to the treatment method in the present invention, the effective dose of the compound of Formula I, the isomer or the pharmaceutically acceptable salt, the isotope substitute, or the pharmaceutical composition thereof above is administered to the patient once every two days or at a longer administration interval.

According to the treatment method in the present invention, the effective dose of the compound of Formula I, the isomer or the pharmaceutically acceptable salt, the isotope substitute, or the pharmaceutical composition thereof above is administered to the patient twice daily.

According to the method for treating the pulmonary hypertension in the present invention, compared with corresponding lung and plasma drug exposures of the treprostinil after intravenous injection administration, after the patient is treated by using the compound of Formula I, the isomer or the pharmaceutically acceptable salt, the isotope substitute, or the pharmaceutical composition thereof, and the treatment method in the present invention, larger pulmonary $C_{max}$ and AUC of the treprostinil may be provided for the patient, which means to provide a larger lung-to-plasma drug exposure ratio.

According to the method for treating the pulmonary hypertension in the present invention, compared with a pulmonary elimination half-life ($t_{1/2}$) of the treprostinil after pulmonary administration, after the patient is treated by using the compound of Formula I, the isomer or the pharmaceutically acceptable salt, the isotope substitute, or the pharmaceutical composition thereof, and the treatment method in the present invention, the treprostinil may have a longer pulmonary elimination half-life in lungs, which greatly increases a retention time of the treprostinil in lungs.

According to the method for treating the pulmonary hypertension in the present invention, compared with a mean area under a pulmonary drug concentration-time curve ($AUC_{0-t}$) of the treprostinil after pulmonary administration, after the patient is treated by using the compound of Formula I, the isomer or the pharmaceutically acceptable salt, the isotope substitute, or the pharmaceutical composition thereof, and the treatment method in the present invention, a larger mean $AUC_{0-t}$ may be provided.

According to the method for treating the pulmonary hypertension in the present invention, compared with the number and severity of side effects experienced by patients when administered with treprostinil, when patients are administered with the compound of Formula I, the isomer or the pharmaceutically acceptable salt, the isotope substitute, or the pharmaceutical composition thereof in the present invention to treat PH, PAH, PPH, ILD-PH, CTEPH and IPF, the number and the severity of side effects experienced by patients are reduced. In some embodiments, the side effect refers to cough reaction of patients, and compared with the frequency and/or severity of cough reaction experienced by patients administered with treprostinil, the frequency and/or severity of cough reaction experienced by patients administered with the compound are reduced.

In an eighth aspect, the present invention provides a method for preparing the compound of Formula I above, which comprises the following step of:

reacting a compound of Formula II with a compound of Formula III to obtain the compound of Formula I,

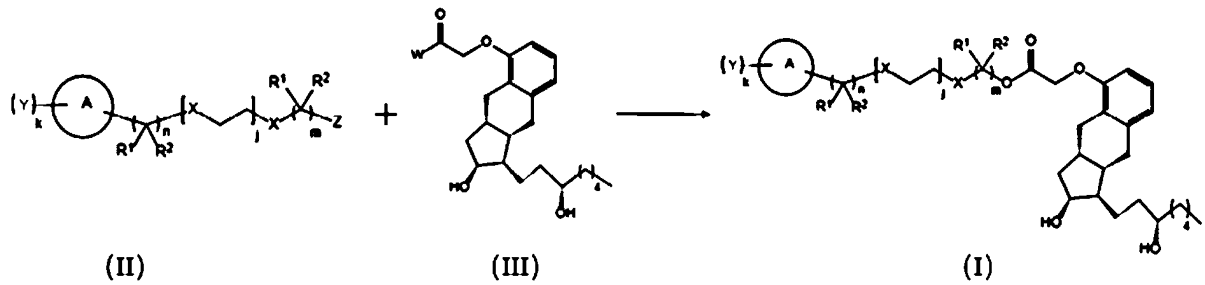

(II) (III) (I)

wherein, W is -OH, -Cl or -OC(O)$R_c$, and $R_c$ is $C_1$-$C_5$ alkyl;
wherein, Z is selected from -OH, -Cl, -Br and -I; and Y, A, $R^1$, $R^2$, X, n, j, m and k are as defined above.

When W is -OH and Z is selected from -Cl, -Br and -I, the compound of Formula (II) is reacted with the compound of Formula (III) in an inert solvent in the presence of a base to obtain the compound of Formula I. Preferably, the base is an organic base or an inorganic base, wherein the organic base is selected from 1,8-diazabicyclo[5.4.0]undec-7-ene (DBU), N,N-diisopropylethylamine and/or diisopropylamine; and the inorganic base is selected from alkaline earth metal carbonate or hydroxide; the inert solvent is selected from ethyl acetate, N,N'-dimethylformamide, tetrahydrofuran, benzene, toluene, dioxane, dichloromethane, acetonitrile and/or polyhalogenated aliphatic hydrocarbon; the reaction is carried out at a temperature ranging from -20°C to 70°C, preferably 45°C to 65°C, for 1~8 hours, preferably 2~5 hours; and when Z is -Cl or -Br, the reaction is carried out in the presence of the alkaline earth metal carbonate, such as potassium carbonate.

When W is -OH and Z is -OH, the compound of Formula (II) is reacted with the compound of Formula (III) in an inert solvent in the presence of a dehydrating agent and a catalyst to obtain the compound of Formula I. Preferably, the dehydrating agent is selected from dicyclohexylcarbodiimide or N'-(3-dimethylaminopropyl)-N-ethylcarbodiimide hydrochloride; the catalyst is selected from N,N-dimethylaminopyridine, sulfuric acid, sulfonic acid, hydrofluoric acid, phosphoric acid, toluenesulfonic acid, polystyrene sulfonate, heteropoly acid, zeolite, metal oxide, graphene oxide or a combination thereof; the inert solvent is selected from N,N-dimethylformamide, tetrahydrofuran, benzene, toluene, dioxane, dichloromethane, acetonitrile and/or polyhalogenated aliphatic hydrocarbon; and the reaction is carried out at a temperature ranging from -20°C to 70°C for 30 minutes to 48 hours.

When W is - Cl and Z is -OH, the compound of Formula (II) is reacted with the compound of Formula (III) in an inert solvent in the presence of an organic base to obtain the compound of Formula I. Preferably, the organic base is selected from N,N-dimethylaminopyridine, triethylamine and/or pyridine; the inert solvent is selected from DMF, tetrahydrofuran, benzene, toluene, dioxane and/or halogenated aliphatic hydrocarbon; and the reaction is carried out at a temperature ranging from -20°C to 70°C for 30 minutes to 24 hours, preferably 2 hours to 12 hours.

When W is -OC(O)$R_c$ and Z is -OH, the compound of Formula (II) reacts with the compound of Formula (III) in an inert solvent in the presence of a catalyst to obtain the compound of Formula I. Preferably, the catalyst is selected from N,N-dimethylaminopyridine, an alkali metal compound catalyst, a Lewis acid catalyst, an alkaline earth metal alkoxide catalyst and/or an organotitanium catalyst; the alkali metal compound catalyst is selected from NaOH, KOH, $Li_2CO_3$, $Na_2CO_3$, $NaHCO_3$, $K_2CO_3$, $Na_2WO_4$ or a combination thereof; the Lewis acid catalyst is selected from $AlCl_3$, $ZnCl_2$, $SnCl_4$, $TiCl_4$, $FeCl_3$, $MgCl_2$, $Pb(OAc)_2$, $Zn(OAc)_2$, $Al_2O(OAc)_4$ or a combination thereof; the alkaline earth metal alkoxide catalyst is selected from sodium methoxide, sodium ethoxide, magnesium methoxide or a combination thereof; the organotitanium catalyst is selected from butyl titanate $Ti(OC_4H_9)_4$, titanocene dichloride ($Cp_2TiCl_2$) or a combination thereof; the inert solvent is selected from N,N-dimethylformamide, tetrahydrofuran, benzene, toluene, dioxane, dichloromethane, acetonitrile and/or polyhalogenated aliphatic hydrocarbon; and the reaction is carried out at a temperature ranging from -20°C to 70°C for 30 minutes to 24 hours, preferably 2 hours to 12 hours.

**Explanations of terms:**

Unless stated to the contrary, the terms used in the specification and the claims have the following meanings.

The term "alkyl" refers to a saturated aliphatic hydrocarbon group, which is a linear or branched group containing 1 to 20 carbon atoms (such as 1, 2, 3, 4, 5, 6, 7, 8, 9, 10, 11, 12, 13, 14, 15, 16, 17, 18, 19 or 20 carbon atoms). The alkyl may be substituted or unsubstituted, and when the alkyl is substituted, the substituent(s) may be attached at any available point of attachment, and the substituent is preferably one or more of the following groups independently selected from alkyl, alkenyl, alkynyl, alkoxy, alkylthio, alkylamino, halogen, sulfhydryl, hydroxyl, nitro, cyano, cycloalkyl, heterocyclic alkyl, aryl, heteroaryl, cycloalkoxy, heterocycloalkoxy, cycloalkylthio, heterocycloalkylthio, oxo, carboxyl and a carboxylate

group.

The term "alkenyl" refers to a linear or branched carbon chain containing one or more carbon-carbon double bonds and 2 to 20 carbon atoms (such as 2, 3, 4, 5, 6, 7, 8, 9, 10, 11, 12, 13, 14, 15, 16, 17, 18, 19 or 20 carbon atoms).

The term "alkynyl" refers to a linear or branched carbon chain containing one or more carbon-carbon triple bonds and 2 to 20 carbon atoms (such as 2, 3, 4, 5, 6, 7, 8, 9, 10, 11, 12, 13, 14, 15, 16, 17, 18, 19 or 20 carbon atoms).

The term "alkylene" refers to a saturated linear or branched aliphatic hydrocarbon group, which has 2 residues derived by removing two hydrogen atoms from the same carbon atom or different carbon atoms of the parent alkane, and is preferably a linear or branched group containing 1 to 10 carbon atoms (such as 1, 2, 3, 4, 5, 6, 7, 8, 9 or 10 carbon atoms). When the alkylene is substituted, the substituent(s) may be attached at any available point of attachment.

The term "cycloalkyl" refers to a saturated or partially unsaturated monocyclic or polycyclic substituent, and the cycloalkyl contains 3 to 12 carbon atoms, preferably 3 to 6 carbon atoms. Nonrestrictive examples of monocyclic cycloalkyl comprise cyclopropyl, cyclobutyl, cyclopentyl, cyclopentenyl, cyclohexyl, cyclohexenyl, cyclohexadienyl, cycloheptyl, cyclohepttrienyl, cyclooctyl, and the like; and polycyclic cycloalkyl comprises spiro, fused and bridged cycloalkyl.

The cycloalkyl may be fused on a ring of aryl, heteroaryl or heterocyclic alkyl, wherein the ring linked to the parent structure is the cycloalkyl. The cycloalkyl may be optionally substituted or unsubstituted.

The term "aryl" refers to a 6- to 14-membered full-carbon monocyclic or fused polycyclic (for example, a ring conjugated with a pair of adjacent carbon atoms) group, preferably a 6- to 10-membered ring, and examples of the aryl comprise, but are not limited to, phenyl, naphthyl, tetrahydronaphthyl, indene and indane.

The aryl may be fused on a ring of heteroaryl, heterocyclyl or cycloalkyl, wherein the ring linked to the parent structure is the aryl ring. The aryl may be optionally substituted or unsubstituted. Nonrestrictive examples of the fused aryl ring comprise:

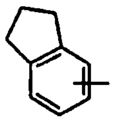 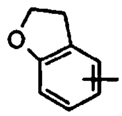 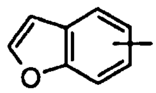 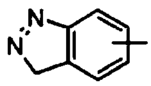 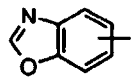 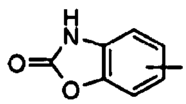 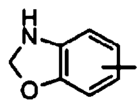

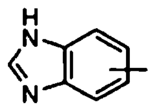 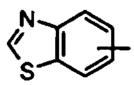 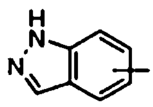 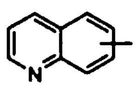 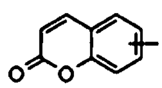 and 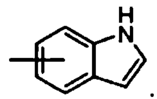 .

The term "heteroaryl" refers to a heteroaromatic system containing 1 to 4 heteroatoms and 5 to 14 ring atoms, wherein the heteroatom is selected from oxygen, sulfur and nitrogen. The heteroaryl is 5-12 membered, more preferably 6-10 membered, and most preferably 8-10 membered. The heteroaryl ring may be fused on the ring of aryl, heterocyclyl or cycloalkyl, wherein the ring linked to the parent structure is the heteroaryl ring. Nonrestrictive examples of the heteroaryl comprise:

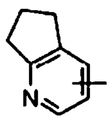 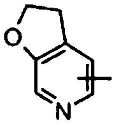 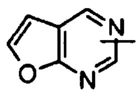 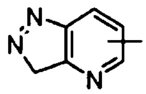 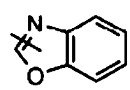 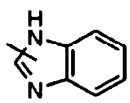 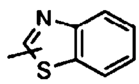

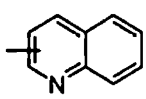 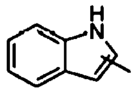 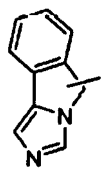 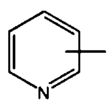 .

The heteroaryl may be substituted or unsubstituted.

The term "substitution" refers to linking to other substituents of a structural part at any acceptable position of the structural part. Unless otherwise specified, the structural part may be bonded through carbon, nitrogen, oxygen, sulfur or any other acceptable atom. The substituent is preferably one or more of the following groups, which are independently selected from alkyl, alkenyl, alkynyl, alkoxy, alkylthio, alkylamino, halogen, sulfhydryl, hydroxyl, nitro, $-ONO_2$, cyano, cycloalkyl, heterocyclyl, aryl, heteroaryl, cycloalkoxy, heterocycloalkoxy, cycloalkylthio, heterocycloalkylthio, oxo, carboxyl or a carboxylate group.

The term "optional" or "optionally" means that the subsequently described event or environment may, but does

not have to, occur, and the description comprises an occasion in which the event or environment occurs or does not occur. For example, the "heterocyclic group optionally substituted by alkyl" means that the alkyl may, but does not have to, exist, and the description comprises the case that the heterocyclic group is substituted by the alkyl and the case that the heterocyclic group is not substituted by the alkyl.

In the chemical structure of the compound in the present disclosure,

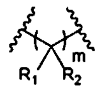

means that there may be a plurality of optionally substituted carbon units, but each carbon unit is not necessarily the same repetitive unit.

In the chemical structure of the compound in the present disclosure,

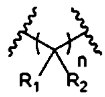

means that there may be a plurality of optionally substituted carbon units, but each carbon unit is not necessarily the same repetitive unit.

In the chemical structure of the compound in the present disclosure, the bond '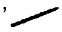' does not specify the configuration, i.e., the bond '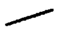'may be '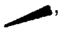' or '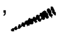', or contains both the two configurations '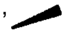' and '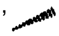' at the same time. In the chemical structure of the compound in the present disclosure, the bond '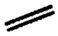' does not specify the configuration, i.e., the bond may have a Z configuration or an E configuration, or contains both the two configurations at the same time.

Although not all the structural formulas above are drawn into specific isomeric forms for simplicity, the present disclosure may contain all isomers, such as a tautomer, a rotamer, a geometric isomer, a diastereomer, a racemate and an enantiomer.

The compound provided by the present disclosure may exist in the form of a pharmaceutically acceptable salt, and the pharmaceutically acceptable salt is selected from: an alkali metal salt such as sodium salt or potassium salt; an alkaline earth metal salt such as calcium salt; an organic alkali salt such as diethylamine salt, diethanolamine salt, meglumine salt, piperazine salt or choline salt; an inorganic acid salt such as hydrochloride, sulfate, nitrate, phosphate, hydrofluoride or hydrobromide; and an organic acid salt such as acetate, trifluoroacetate, naphthoate, benzoate, tartrate, lactate, citrate, fumarate, maleate, malate, oxalate, succinate, methanesulfonate, ethanesulfonate, benzenesulfonate, p-toluenesulfonate, naphthalenesulfonate or camphorsulfonate.

Compared with the prior art, the present invention has the following beneficial effects.

1. The compound of Formula I provided by the present invention exhibits improved metabolic properties, and an innovative side chain structure makes the compound of Formula I have a strong cell membrane affinity, resulting in unique diffusion microkinetic properties, following pulmonary administration, the compound can remain locally in lungs for an extended duration and is slowly converted into treprostinil at an effective concentration, which significantly increases the retention time and elimination half-life of treprostinil in lungs, thereby greatly prolonging the duration of drug action.
2. After the pulmonary administration of the compound of Formula I provided by the present invention, a significantly increased lung-to-plasma drug concentration ratio of treprostinil and a greater lung exposure of treprostinil are achieved.
3. The compound of Formula I provided by the present invention can greatly reduce an administration dosage, provide a lower administration frequency and a broader therapeutic window, and significantly reduce systematic side effects and local adverse reaction of lungs, thereby greatly improving the drug safety and patient tolerability to the drug.
4. The compound of Formula I provided by the present invention has better physical and chemical properties and stability in solvent, so that the compound is easier to be prepared into a drug, and the industrial production cost is reduced, so as to be expected to reduce patients' treatment costs, thereby providing a new treatment choice for patients with pulmonary hypertension.

DESCRIPTION OF THE DRAWINGS

FIG. 1(a) shows hydrolysis conversion rates, mediated by porcine esterase, of the alkyl ester prodrug compound (R-01) with 10 carbon atoms and ether bond-containing alkyl ester prodrug compounds (R-05, R-06 and R-08) with the same number of carbon atoms.

FIG. 1(b) shows hydrolysis conversion rates, mediated by porcine esterase, of the alkyl ester prodrug compounds containing a carboxylic ester bond (R-09, R-10 and R-11).

FIG. 1(c) shows hydrolysis conversion rates, mediated by the porcine esterase, of compounds (Ia-1, Ia-2 and Ia-3) of the present invention and control compounds (R-12 and C16-TR).

FIG. 2(a) shows percentage contents of compound Ia-2 in solution samples with different pH values after light irradiation for 5 days or 10 days, wherein the ratios in brackets on an abscissa represent the ethanol-to-water ratios in the sample solutions.

FIG. 2(b) shows the percentage contents of compound Ia-2 in the solution samples with different pH values after storage at 40°C for 10 days or 30 days, wherein the ratios in brackets on an abscissa represent the ethanol-to-water ratios in the sample solutions.

FIG. 2(c) shows percentage contents of compound Ia-2 in the solution samples with different pH values after storage at 60°C for 10 days or 30 days, wherein the ratios in brackets on an abscissa represent the ethanol-to-water ratios in the sample solutions.

FIG. 2(d) shows percentage contents of ethyl ester impurities of compound Ia-2 in the solutions with different pH values under light irradiation.

FIG. 2(e) shows percentage contents of the ethyl ester impurities of compound Ia-2 in solutions with different pH values under high-temperature conditions.

FIG. 3 shows in-vitro agonist activities of TRE and treprostinil derivatives (Ia-1, Ia-2 and Ia-3) on IP receptor.

FIG. 4 shows competitive inhibitory activities of TRE and treprostinil derivatives (la-1, la-2 and Ia-3) on binding of $^3$H-PGE2 to EP2 receptor, which reflects the binding affinities of the compounds to EP2 receptor.

FIG. 5 shows the percentage inhibition of the increase in right ventricular systolic pressure (RVSP) induced by U46619 at various time points after airway nebulized administration of the compounds of the present invention (Ia-1, Ia-2 and Ia-3) and the control compounds (TRE, R-11, R-12, C16-TR). The RVSP values at 16 hours after administration of compound R-12 and at 48 hours after administration of compound Ia-2 had increased above the basal pressure, indicating the absence of an antihypertensive effect; therefore, the corresponding data are not shown in the figure.

FIG. 6 shows the percentage inhibition of the increase in RVSP induced by U46619 at 1 hour after low-dose airway nebulized administration of compounds of the present invention (Ia-1, Ia-2 and Ia-3) and the control compound (C16-TR).

EMBODIMENT

The technical solutions of the present invention are described clearly and completely hereinafter with reference to the embodiments, and the embodiments described are only used to illustrate the technical solutions of the present invention, and are not intended to limit the present invention. Based on the embodiments in the present invention, all other embodiments obtained by those of ordinary skills in the art without going through any creative work should fall within the scope of protection of the present invention. Unless otherwise specified, the reagents and raw materials used in the present invention are commercially available.

**Preparation examples of compounds**

The structure sof compounds are determined by nuclear magnetic resonance ($^1$H NMR) or liquid chromatography-mass spectrometry (LC-MS).

The liquid chromatography-mass spectrometry instrument (LC-MS) is Agilent 6120; and the nuclear magnetic resonance instrument ($^1$H NMR) is Bruker Avance-400, wherein the nuclear magnetic resonance ($^1$H NMR) shift ($\delta$) is given in parts per million (ppm), the determination solvent is $d_6$-DMSO, the internal standard is tetramethylsilane (TMS), and the chemical shift is given in $10^{-6}$ (ppm).

The term "room temperature" in the present invention refers to a temperature from 10°C to 30°C.

Comparative examples. Preparation of compounds R-01, R-03, R-05, R-06, and R-08 to R-12

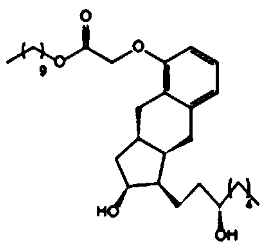

Compound R-01

Treprostinil (500.6 mg, 1.282 mmol) was dissolved in anhydrous DMF (8 mL). To this solution was added anhydrous potassium carbonate (442.4 mg, 3.201 mmol) and 1-bromodecane (452 mg, 2.044 mmol). The mixture was stirred to react at 60°C for 3 hours. After the reaction was completed, the reaction system was cooled to room temperature, diluted with ethyl acetate and washed with water. The organic phase was dried over anhydrous sodium sulfate and concentrated under reduced pressure to afford a crude product. Purification by silica gel column chromatography (ethyl acetate/n-hexane) yielded compound R-01 (540.0 mg, 1.018 mmol), with a yield of 79.4%. (MS)ESI $[M+Na]^+$: 553.40. HNMR (DMSO-$d_6$, 400MHz) δ0.85 (6H, m); δ0.99-1.10 (2H, m); δ1.10~1.56 (25H, m); δ1.56 (3H, m); δ1.75 (1H, m); δ1.96~2.11 (2H, m); δ2.37 (2H, m); δ2.71 (2H, m); δ3.38 (1H, m); δ3.47 (1H, m); δ4.09 (2H, t); δ4.22 (1H, d); δ4.48 (1H, d); δ4.75 (2H, s); δ6.69~6.76 (2H, d×d); δ7.02 (1H, t).

With reference to the preparation method of compound R-01, compounds R-03, R-05, R-06, and R-08 to R-12 were synthesized. Treprostinil (500.6 mg, 1.282 mmol) was dissolved in anhydrous DMF (8 mL). Anhydrous potassium carbonate (442.4 mg, 3.201 mmol) and the appropriate side-chain compound (0.9~2.0 times the molar amount of treprostinil) were added to the solution. The mixture was stirred to react at 60°C for 3 hours. After the reaction was completed, the reaction system was cooled to room temperature, diluted with ethyl acetate and washed with water. The organic phase was dried over anhydrous sodium sulfate and concentrated undder recduced pressure to afford a crude product, which was purified by silica gel column chromatography (ethyl acetate/n-hexane) to yield the respective. Structure, (MS)ESI and NMR data of each control compound were as follows:

| Serial number | Structure of compound | (MS)ESI [M+Na]+ | $^1$HNMR |
|---|---|---|---|
| Compound R-03 | 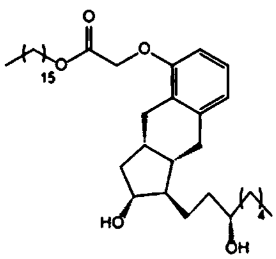 | 637.4 | $^1$HNMR (DMSO-$d_6$, 400MHz) δ0.85 (6H, m); δ0.94-1.68 (42H, m); δ1.76 (1H, m); δ1.92 (1H, m); δ2.12 (1H, m); δ2.43 (2H, m); δ2.68 (2H, m); δ3.34 (1H, m); δ3.46 (1H, m); δ4.10 (2H, t); δ4.22 (1H, d); δ4.49 (1H, d); δ4.75 (2H, s); δ6.75 (2H, d×d); δ7.02 (1H, t) |
| Compound R-05 | 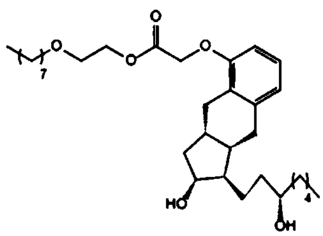 | 569.3 | $^1$HNMR (DMSO-$d_6$, 400MHz) δ0.85 (6H, m); δ0.98-1.48 (25H, m); δ1.61 (1H, m); δ1.76 (1H, m); δ1.96 (1H, m); δ2.11 (1H, m); δ2.41 (2H, m); δ2.70 (2H, m); δ3.37 (3H, m); δ3.48 (1H, m); δ3.57 (2H, t); δ4.24 (3H, m); δ4.48 (1H, d); δ4.76 (2H, s); δ6.74 (2H, d×d); δ7.03 (1H, t) |
| Compound R-06 | 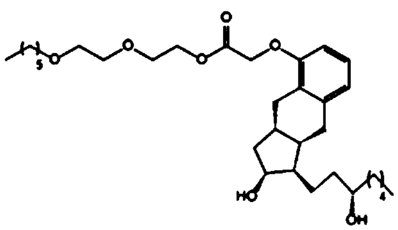 | 585.4 | $^1$HNMR (DMSO-$d_6$, 400MHz) δ0.86 (6H, m); δ0.96-1.48 (21H, m); δ1.61 (1H, m); δ1.77 (1H, m); δ1.96 (1H, m); δ2.12 (1H, m); 82.41 (2H, m); δ2.69 (2H, m); δ3.37 (3H, m); δ3.44~3.53 (5H, m); δ3.63 (2H, t); δ4.24 (3H, d); δ4.48 (1H, d); δ4.75 (2H, s); δ6.74 (2H, d×d); δ7.03 (1H, t) |

(continued)

| Serial number | Structure of compound | (MS)ESI [M+Na]+ | 1HNMR |
|---|---|---|---|
| Compound R-08 | 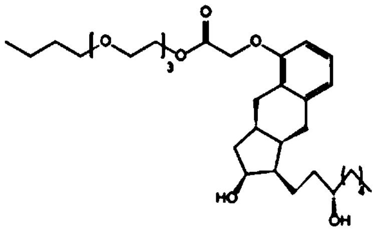 | 601.3 | 1HNMR (DMSO-$d_6$, 400MHz) δ0.87 (6H, m); δ0.96~1.47 (17H, m); δ1.60 (1H, m); δ1.77 (1H, m); δ1.96 (1H, m); δ2.13 (1H, m); δ2.42 (2H, m); δ2.70 (2H, m); δ3.36 (4H, t); δ3.45~3.54 (9H, m); δ3.63 (2H, t); δ4.24 (3H, m); δ4.48 (1H, d); δ4.77 (2H, s); δ6.74 (2H, d×d); δ7.04 (1H, t) |
| Compound R-09 | 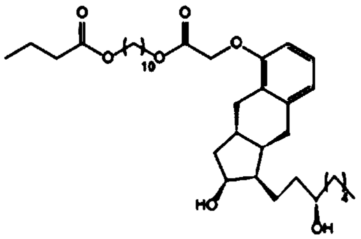 | 639.4 | 1HNMR (DMSO-$d_6$, 400MHz) δ0.86 (6H, m); δ0.99~1.63 (32H, m); δ1.76 (1H, m); δ1.94 (1H, m); δ2.11 (1H, m); δ2.25 (2H, d); δ2.41 (2H, m); δ2.71 (2H, m); δ3.34 (1H, m); δ3.48 (1H, m); δ4.00 (2H, m); δ4.10 (2H, t); δ4.24 (1H, d); δ4.50 (1H, d) δ4.75 (2H, s); δ6.69~6.78 (2H, d×d); δ7.03 (1H, t) |
| Compound R-10 | 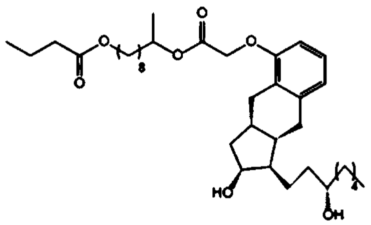 | 629.4 | 1HNMR (DMSO-$d_6$, 400MHz) δ0.86 (6H, m); δ0.99~1.63 (33H, m); δ1.76 (1H, m); δ1.95 (1H, m); δ2.11 (1H, m); δ2.25 (2H, d); δ2.43 (2H, m); δ2.66 (2H, m); δ3.39 (1H, m) δ3.47 (1H, m); δ4.00 (2H, m); δ4.23 (1H, d); δ4.49 (1H, d); δ4.74 (2H, s); δ4.87 (1H, m); δ6.67~6.76 (2H, d×d); δ7.01 (1H, t) |
| Compound R-11 | 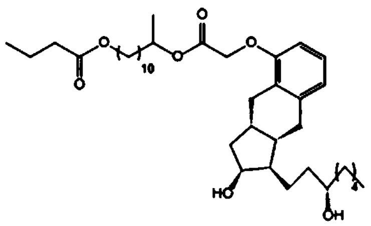 | 667.4 | 1HNMR (DMSO-$d_6$, 400MHz) δ0.86 (6H, m); δ0.96~1.85 (37H, m); δ1.76 (1H, m); δ1.95 (1H, m); δ2.12 (1H, m); δ2.26 (2H, t); δ2.42 (2H, m); δ2.70 (2H, m); δ3.38 (1H, m); δ3.48 (1H, m);δ4.01 (2H, m); δ4.24 (1H, d); δ4.48 (1H, dd); δ4.72 (2H, s); δ4.88 (1H, m) δ6.67 (1H, d); δ6.76 (1H, d); δ7.03 (1H, t) |
| Compound R-12 | 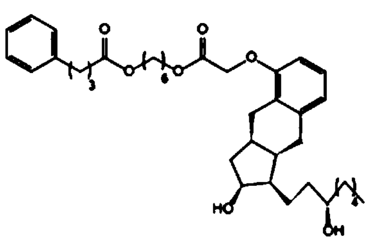 | 659.4 | 1HNMR (DMSO-$d_6$, 400MHz) δ0.86 (3H, t); δ0.95~1.75 (25H, m); δ1.95 (1H, m); δ2.13 (1H, m); δ2.29 (2H, m); δ2.42 (2H, m); δ2.58 (2H, t); δ2.70 (2H, m); δ3.37 (1H, m); δ3.48 (1H, m); δ4.01 (2H, t); δ4.10 (2H, t); δ4.23 (1H, d); δ4.49 (1H, d); δ4.75 (2H, s); δ6.73 (2H, m); δ7.03 (1H, t); δ7.18 (3H, m); δ7.27 (2H, t) |

Preparation Example 1 Preparation of compound Ia-1

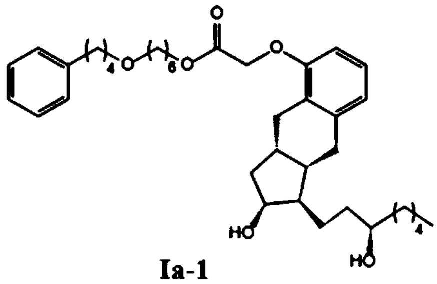

Ia-1

Treprostinil (504.0 mg, 1.291 mmol) was dissolved in anhydrous DMF (8 mL).To this solution were added anhydrous potassium carbonate (445.73 mg, 3.225 mmol) and [4-[(6-bromohexyl)oxy)]butyl]-benzene (640 mg, 2.051 mmol). The mixture was stirred to react at 60°C for 3 hours. After the reaction was completed, the reaction system was cooled to room temperature, diluted with ethyl acetate and washed with water. The organic phase was dried over anhydrous sodium sulfate and concentrated to obtain a crude product, and the crude product was purified by silica gel column chromatography (ethyl acetate/n-hexane) to obtain the compound Ia-1 (600 mg, 0.964 mmol), with a yield of 74.7%. (MS) ESI [M+H]+: 623.4. HNMR (DMSO-d6, 400MHz) δ0.87 (3H, t); δ1.04~1.62 (26H, m); δ1.76 (1H, m); δ1.95 (1H, m); δ2.11 (1H, m); δ2.42 (2H, m); δ2.57 (2H, t); δ2.71 (2H, m); δ3.30 (2H, m); δ3.36 (3H, m); δ3.47 (1H, m); δ4.11 (2H, t); δ4.23 (1H, d); δ4.49 (1H, d); δ4.75 (2H, s); δ6.69~6.75 (2H, d×d); δ7.02 (1H, t); δ7.10~7.30 (5H, m).

**Preparation Example 2 Preparation of compound Ia-2**

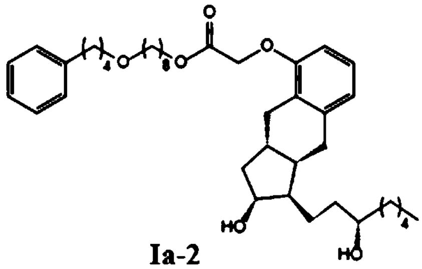

Ia-2

Treprostinil (503.8 mg, 1.290 mmol) was dissolved in anhydrous DMF (8 mL). To this solution were added anhydrous potassium carbonate (443.3 mg, 3.207 mmol) and [4-[(8-bromohexyl)oxy)]butyl]-benzene (680 mg, 1.999 mmol). The mixture was stirred to react at 60°C for 3 hours. After the reaction was completed, the reaction system was cooled to room temperature, diluted with ethyl acetate and washed with water, an organic phase was dried over anhydrous sodium sulfate and concentrated to obtain a crude product, and the crude product was purified by silica gel column chromatography (ethyl acetate/n-hexane) to obtain the compound Ia-2 (550 mg, 0.846 mmol), with a yield of 65.5%. (MS) ESI [M+Na]+: 673.4. HNMR (DMSO-d6, 400MHz) δ0.86 (3H, t); δ0.96~1.67 (30H, m); δ1.76 (1H, m); δ1.95 (1H, m); δ2.11 (1H, m); δ2.41 (2H, m); δ2.58 (2H, t); δ2.68 (2H, m); δ3.30 (2H, m); δ3.36 (3H, m); δ3.47 (1H, m); δ4.09 (2H, t); δ4.23 (1H, d); δ4.49 (1H, d); δ4.75 (2H, s); δ6.73 (2H, dd); δ7.03 (1H, t); δ7.18 (3H, m); δ7.27 (2H, t).

**Preparation Example 3 Preparation of compound Ia-3**

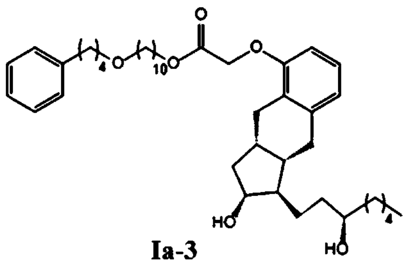

Ia-3

Treprostinil (505.1mg, 1.293 mmol) was dissolved in anhydrous DMF (8 mL).To this solution were added

anhydrous potassium carbonate (448.4 mg, 3.244 mmol) and [4-[(10-bromohexyl)oxy)]butyl]-benzene (700 mg, 1.895 mmol). The mixture was stirred to react at 60°C for 3 hours. After the reaction was completed, the reaction system was cooled to room temperature, diluted with ethyl acetate and washed with water, an organic phase was dried over anhydrous sodium sulfate and concentrated to obtain a crude product, and the crude product was purified by silica gel column chromatography (ethyl acetate/n-hexane) to obtain the compound Ia-3 (600 mg, 0.884 mmol), with a yield of 68.4%. (MS) ESI [M+Na]+: 701.4. HNMR (DMSO-d6, 400MHz) δ0.86 (3H, t); δ0.96~1.67 (34H, m); δ1.76 (1H, m); δ1.95 (1H, m); δ2.10 (1H, m); δ2.42 (2H, m); δ2.58 (2H, t); δ2.71 (2H, m); δ3.30 (2H, m); δ3.36 (3H, m); δ3.48 (1H, m); δ4.09 (2H, t); δ4.24 (1H, d); δ4.48 (1H, d); δ4.75 (2H, s); δ6.73 (2H, dd); δ7.03 (1H, t); δ7.19 (3H, m); δ7.25 (2H, t).

The following compounds were synthesized by similar procedures as described above:

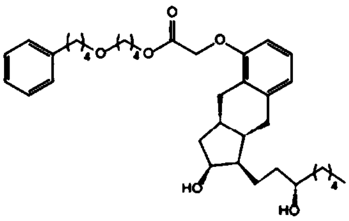
Ia-4 ,

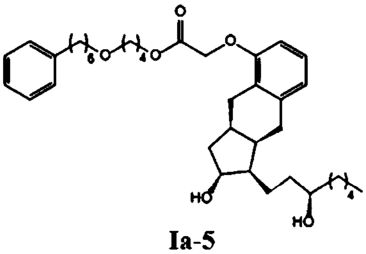
Ia-5 ,

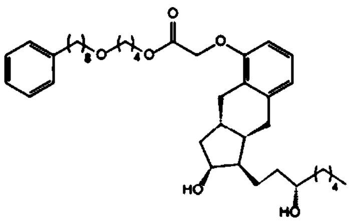
Ia-6 ,

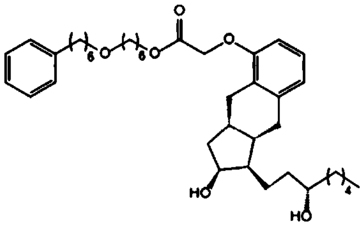
Ia-7 ,

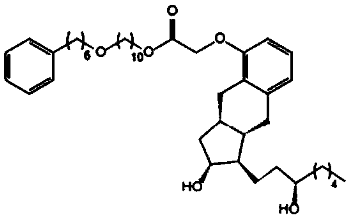
Ia-8 ,

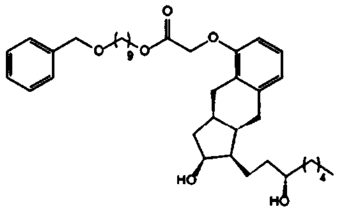
Ia-9 ,

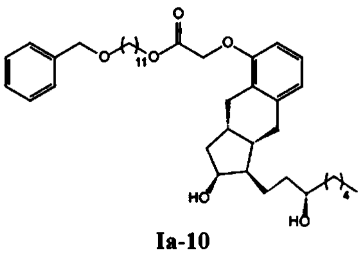
Ia-10 ,

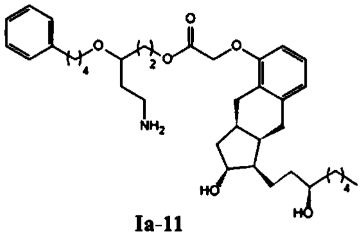
Ia-11 ,

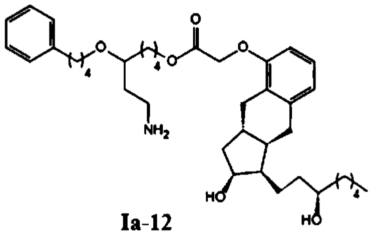
Ia-12 ,

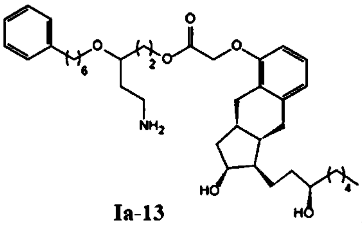
Ia-13 ,

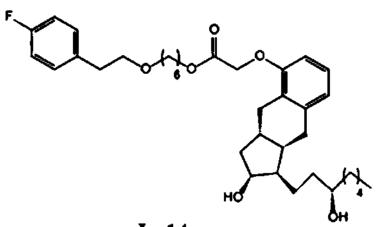
Ia-14 ,

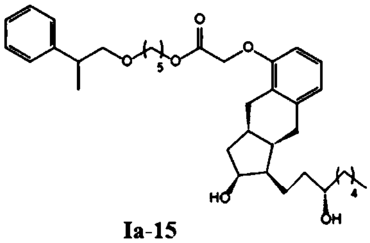
Ia-15 ,

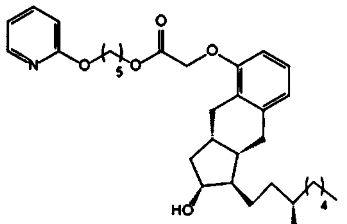
Ia-16 , and

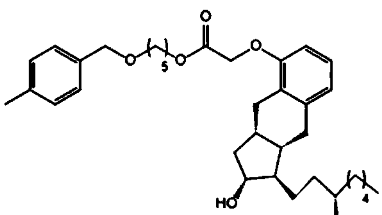
Ia-17 .

**Preparation** Example 4 Preparation of compound Ia-18

(1) Preparation of intermediate compound (2-((6-bromohexyl)oxy)ethoxy)methyl)benzene

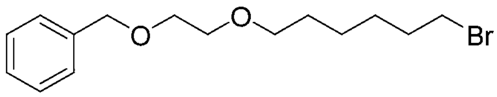

Sodium hydride (0.984 g, 0.0246 mol) was dissolved in anhydrous THF (30 mL), slowly added with 1,6-dibromohexane (12 g, 0.0492 mol), stirred under the protection of $N_2$ for 30 minutes, then added with 2-benzyloxy ethanol (2.5 g, 0.0164 mol), and refluxed at 75°C overnight. After cooling, the mixture was added with water to quench the sodium hydride, added with ethyl acetate for dilution and washed with saturated salt water, an organic phase was dried over anhydrous sodium sulfate and concentrated to obtain a crude product, and the crude product was purified by silica gel column chromatography (ethyl acetate: n-hexane=1: 90) to obtain the compound (2-((6-bromohexyl)oxy)ethoxy)methyl) benzene (2.6963 g, 0.00855 mol), with a yield of 52.2%. $^1$H NMR (500 MHz, $CDCl_3$): δ7.37-7.27 (m, 5H), 4.58 (s, 2H), 3.61 (s, 4H), 3.47 (t, J = 6.6 Hz, 2H), 3.40 (t, J = 6.8 Hz, 2H), 1.86 (p, J = 6.9 Hz, 2H), 1.61 (p, J = 6.8 Hz, 2H), 1.50-1.33 (m, 4H).

(2) Preparation of compound Ia-18

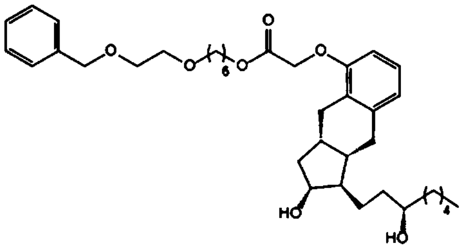

Compound Ia-18

Treprostinil (522.20 mg, 1.337 mmol) was dissolved in anhydrous DMF (5 mL), and added with anhydrous potassium carbonate (501.71 mg, 3.630 mmol) and (2-((6-bromohexyl)oxy)ethoxy)methyl)benzene (479.8 mg, 1.402 mmol). The mixture was stirred to react at 60°C for 3 hours. After the reaction was completed, the reaction system was cooled to room temperature, added with ethyl acetate for dilution and washed with water, an organic phase was dried over anhydrous sodium sulfate and concentrated to obtain a crude product, and the crude product was purified by silica gel column chromatography (ethyl acetate/n-hexane) to obtain the compound Ia-18 (800 mg, 1.281mmol), with a yield of 95.8%. (MS) ESI [M+Na]+: 647.0. $^1$HNMR ($CDCl_3$-d6, 400MHz);δ0.92 (3H, d);δ1.10~1.75 (22H, m); δ1.91 (1H, m); δ2.22 (2H, m); δ2.35-2.47 (2H, m); δ2.76-2.91 (2H, m); δ3.48 (2H, t); δ3.63 (5H, t); δ3.77 (1H, m); δ4.20 (2H, m); δ4.61 (4H, d); δ6.63 (1H, t); δ6.83 (1H, d); δ7.07 (1H, m); δ7.28-7.36 (5H, m)

**Preparation Example 5 Preparation of compound Ia-19**

(1) Preparation of intermediate compound 2-((2-((6-bromohexyl)oxy)ethoxy)methyl)-1,3-dichlorobenzene

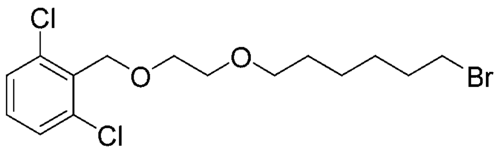

Sodium hydride (0.678 g, 0.0169 mol) was dissolved in anhydrous THF (30 mL), slowly added with 1,6-dibromohexane (8.27 g, 0.0339 mol), stirred under the protection of $N_2$ for 30 minutes, then added with 2-((2,6-dichlorobenzyl)oxy)ethanol (2.5 g, 0.0113 mol), and refluxed at 75°C overnight. After cooling, the mixture was added

with water to quench the sodium hydride, added with ethyl acetate for dilution and washed with saturated salt water, an organic phase was dried over anhydrous sodium sulfate and concentrated to obtain a crude product, and the crude product was purified by silica gel column chromatography (ethyl acetate: n-hexane=1: 200) to obtain the compound 2-((2-((6-bromohexyl)oxy)ethoxy)methyl)-1,3-dichlorobenzene (2.8103 g, 0.00732 mol), with a yield of 64.7%. $^1$H NMR (500 MHz, CDCl3) : δ7.31 (d, J = 8.0 Hz, 2H), 7.18 (dd, J = 8.5, 7.5 Hz, 1H), 4.83 (s, 2H), 3.70 (dd, J = 5.9, 3.9 Hz, 2H), 3.61 (dd, J = 5.8, 4.0 Hz, 2H), 3.46 (t, J = 6.6 Hz, 2H), 3.40 (t, J = 6.9 Hz, 2H), 1.85 (p, J = 7.0 Hz, 2H), 1.62-1.56 (m, 2H), 1.48-1.35 (m, 4H).

(2) Preparation of compound Ia-19

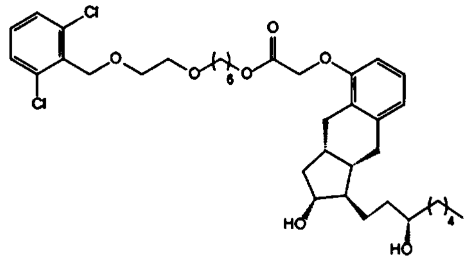

Compound Ia-19

Treprostinil (518.57 mg, 1.328 mmol) was dissolved in anhydrous DMF (5 mL), and added with anhydrous potassium carbonate (278.92 mg, 2.018 mmol) and 2-((2-((6-bromohexyl)oxy)ethoxy)methyl)-1,3-dichlorobenzene (457.16 mg, 1.115 mmol). The mixture was stirred to react at 60°C for 3 hours. After the reaction was completed, the reaction system was cooled to room temperature, added with ethyl acetate for dilution and washed with water, an organic phase was dried over anhydrous sodium sulfate and concentrated to obtain a crude product, and the crude product was purified by silica gel column chromatography (ethyl acetate/n-hexane) to obtain the compound Ia-19 (800 mg, 1.155mmol), with a yield of 86.9%. (MS) ESI [M+Na]+: 714.8. HNMR (CDCl3-d6, 400MHz) δ0.92 (3H, t) δ1.28-1.68 (22H, m); δ1.90 (1H, m); δ2.23 (2H, m); δ2.49 (1H, m); δ2.58 (1H, m); δ2.77 (1H, m); δ2.89 (1H, m); δ3.47 (2H, t); δ3.62 (3H, m); δ3.75 (3H, m); δ4.20 (2H, t); δ4.63 (2H, s); δ4.85 (2H, ss); δ6.66 (1H, d); δ6.83 (1H, d); δ7.08 (1H, t); δ7.19 (1H, t); δ7.32 (2H, m).

**Experimental examples**

**Experimental Example 1. Study on hydrolysis conversion rate of compounds mediated by porcine esterase**

Under the effect of esterase, the compounds provided by the present invention could be hydrolyzed into the active ingredient treprostinil (TRE). This experiment was designed to evaluate the conversion rates of various compounds with different ester side chain structures into the TRE under the action of esterase.

According to the patent literature CN105848479B, the hydrolysis rate of the alkyl ester prodrugs of TRE under the effect of esterase was related to the carbon chain length of the ester side chain. When the carbon number of the alkyl side chain was 8 or less, the prodrug compound was 100% converted into TRE within 30 minutes under the effect of esterase. When the carbon number was >8, the conversion rate of the prodrug into TRE could be significantly delayed.

**Experiment (a): evaluation of hydrolysis conversion rates of compounds with ether bond-containing alkyl ester side chains**

An aliquot of 20 mg of each control compound R-01, R-05, R-06 or R-08 was weighed. The carbon number of the ester side chain of each compound was 10, wherein the alkyl side chains of R-05, R-06 and R-08 contained 1~3 oxygen atoms respectively. Each compound was dissolved in 20 mL of ethanol to prepare a 1 mg/ml test compound solution for later use. Then, 1 mL of the test compound solution was transferred into 4 mL of PBS aqueous solution to obtain 0.2 mg/mL test compound reaction solution. The reaction solution was added with 20 U (80 μL) of porcine esterase, shaken evenly and then incubated at 38°C. After a period of reaction, samples were taken according to the following time points: 10 minutes, 30 minutes, 1 hour and 2 hours. The conversion of the test compound into TRE was detected. The hydrolysis of each sample was calculated according to measured peak areas of the reactant and the product:

% hydrolysis = peak area of product/(peak area of reactant + peak area of product) * 100%

Results of this experiment are shown in FIG. 1(a). The results showed that the conversion rate of the prodrug compound into TRE could be significantly accelerated after inserting oxygen atoms into the alkyl side chain of the prodrug compound.

**Experiment (b): evaluation of hydrolysis conversion rates of compounds with ester bond-containing alkyl ester side chains**

Hydrolysis conversion rates of control compounds R-09, R-10 and R-11 were detected by the method as described in the experiment (a). Sampling time points were: 20 minutes, 40 minutes, 1 hour and 2 hours. Results of this experiment are shown in FIG. 1(b). The results showed that the hydrolysis of each compound was nearly complete at 40 minutes. When the aliphatic side chain contained a carboxylic ester structure, increasing of carbon number could not prolong the conversion rate of the compound into TRE, and even if a steric hindrance around the ester bond linking the side chain to TRE was increased, the conversion rate was not obviously prolonged.

Experiment (c): evaluation of hydrolysis conversion rates of compounds provided by the present invention and control compounds

Hydrolysis conversion rates of the compounds Ia-1, Ia-2 and Ia-3 of the present invention and the compounds C16-TR, R-09 and R-12 were detected by the method as described in the experiment (a). Sampling time points were: 5 minutes, 15 minutes, 30 minutes, 1 hour and 2 hours. Results of this experiment are shown in FIG. 1(c). The results showed that, the same as C16-TR, the compounds of the present invention could significantly prolong the hydrolysis conversion rates and release TRE slowly and steadily, which also reflected that the compounds of the present invention could increase the pulmonary elimination half-life of treprostinil to some extent. Moreover, with the increase of carbon number in the ester side chains of the compounds of the present invention, the hydrolysis rates slowed down.

Experimental Example 2. Study on stability of treprostinil derivative **in** ethanol

The experimental example was intended to evaluate the stability of the treprostinil derivative in ethanol.

1 mol/L hydrochloric acid and/or sodium hydroxide aqueous solution and distilled water were used to prepare 100 mL solutions with different pH values (3.0, 4.0, 5.0, 6.0, 7.0, 8.0, 9.0 and 10.0) for later use. 640 mg of compound Ia-2 was taken and added with 640 mL of 90% ethanol as a diluent to completely dissolve the compound, and then prepared into 1 mg/mL sample solution 1 for later use.

(1) 40 mL of 90% ethanol, the sample solution 1 and 80 mL of each aqueous solution with different pH value (3.0, 5.0, 7.0 or 9.0) were taken and mixed evenly (ethanol: water =1.5: 1), and filled 2 mL per vial to obtain total 90 vials of samples, and the samples were divided into three groups equally for stability examination under the condition of light-irradiation, high-temperature of 40°C or high-temperature of 60°C. The samples of the two high-temperature stability examination groups were packaged in aluminum foil bags, 10 vials per bag; and the samples of the light-irradiation stability examination group were packaged in transparent bags, 10 vials per bag.

(2) 80 mL of 90% ethanol, the sample solution 1 and 80 mL of each aqueous solution with different pH value (4.0, 6.0, 8.0 or 10.0) were taken and mixed evenly (ethanol: water =2: 1), and then filled, grouped and packaged as above.

After the above samples were placed under conditions as described in Table-1, 15 vials of samples were taken from each group at each detection time point for detection for each pH value solution, comprising: the content of compound, the content of related substances (impurities) and liquid properties (whether there was turbidity, stratification or color change). The content of compound and the content of related substances were calculated by calculating the percentage of area occupied by the main ingredient through an area normalization method.

**Table-1 Placement conditions for stability examination of compound Ia-2**

| pH | High temperature (40°C) | High temperature (60°C) | Light irradiation | Number |
|---|---|---|---|---|
| 3 | 10 days and 30 days | 10 days and 30 days | 5 days and 10 days | Sampling quantity: 60 vials (quantity placed: 90 vials) |
| 4 | 10 days and 30 days | 10 days and 30 days | 5 days and 10 days | Sampling quantity: 60 vials (quantity placed: 90 vials) |
| 5 | 10 days and 30 days | 10 days and 30 days | 5 days and 10 days | Sampling quantity: 60 vials (quantity placed: 90 vials) |

(continued)

| pH | High temperature (40°C) | High temperature (60°C) | Light irradiation | Number |
|---|---|---|---|---|
| 6 | 10 days and 30 days | 10 days and 30 days | 5 days and 10 days | Sampling quantity: 60 vials (quantity placed: 90 vials) |
| 7 | 10 days and 30 days | 10 days and 30 days | 5 days and 10 days | Sampling quantity: 60 vials (quantity placed: 90 vials) |
| 8 | 10 days and 30 days | 10 days and 30 days | 5 days and 10 days | Sampling quantity: 60 vials (quantity placed: 90 vials) |
| 9 | 10 days and 30 days | 10 days and 30 days | 5 days and 10 days | Sampling quantity: 60 vials (quantity placed: 90 vials) |
| 10 | 10 days and 30 days | 10 days and 30 days | 5 days and 10 days | Sampling quantity: 60 vials (quantity placed: 90 vials) |

Specific results of this experiment were as shown in FIG. 2. Results of FIGs. 2a-c showed that the compound provided by the present invention could stably exist in high-concentration ethanol solutions with different pH values (ethanol: water =1.5: 1, ethanol: water = 2.0: 1), and its content was still higher than 95% after placement for 10 days. Even after placement at 40°C for 30 days, the content of compound Ia-2 in each group was still higher than 80%; and after placement at 60°C for 30 days, the content of compound Ia-2 was still higher than 90% in the solution with pH=5.0~9.0, which was a very exciting result.

According to the reports in literatures CN105848479B and Adam J. Praunt, et al. (2021), C16-TR could not be dissolved in HFA propellant when the content of ethanol was lower than 13%, DSPE-PEG2000 had to be added to assist dissolution, and it could not remain soluble during long-term storage (>1 week), that is, it exhibits poor physical stability (there was a precipitation ring at the gas-liquid interface, freely floating insoluble substances were observed, etc.), and generates ester exchange impurities with the alcohol solvent. Surprisingly, the compounds provided in the present invention demonstrated good solubility in ethanol, and could be completely dissolved in 8% ethanol without adding additional cosolvent. In the studies on various influencing factors (light irradiation, high temperature, and strong acid and strong base) in this experimental example, all the compounds of the present invention showed good solubility (all solutions remained clear) and stability. As shown in FIGs. 2d-e, under light irradiation, the ethyl ester impurities formed in each group were less than 1.80% after 10 days of dosage. In the case of high-temperature placement for a long time, about 8.0~9.0% ethyl ester impurities could be formed only under extreme conditions (pH=3, placement at 60°C for 30 days; pH=10, placement at 40°C for 30 days or placement at 60°C more than 10 days), and the ethyl ester impurities were less than 4.0% under other conditions.

Experimental Example 3. Experiment for in-vitro agonist activity of treprostinil derivative on **IP** receptor

Aagonist activities of the compounds (Ia-1, Ia-2 and Ia-3) of the present invention to the IP receptor were tested by FLIPR test for detecting calcium ions, and compared with that of the compound TRE. The specific method is as follows:

a) Preparation of cells:

The IP/HEK293 cell line was provided by WuXi AppTec (Shanghai) Co., Ltd. Well-grown IP/HEK293 cells were taken out from the incubator, the culture solution was aspirated, the cells were washed with D-PBS and digested with trypsin EDTA, and then added with a cell culture solution, and the cells were fully suspended by repeated pipetting. The cells were centrifuged at room temperature, the supernatant was aspirated, a culture medium was added, and the cells were resuspended by repeated pipetting with a sterile pipette. After counting living cells with Vi-CELL™, 20 μL (20,000 cells/well) of cell suspension was added to a 384-well cell culture plate and incubated in a 5% $CO_2$ incubator at 37°C overnight.

b) Preparation of FLIPR reagent:

1 mL of FLIPR test buffer was added with 77 mg of probenecid to prepare 250 mM probenecid solution. After thawing a bottle of 2XFluo-4 DirectTM (F10471), the bottle was added with 10 mL of FLIPR test buffer containing 500 uM AEBSF, and each 10 mL of Fluo-Direct was added with 0.2 mL of probenecid, with a final concentration of 2.5 mM. The mixture was vortexed and then allowed to stand in the dark for more than 5 minutes.

c) Preparation of compounds

Each compound was placed in a water bath at 25°C for 30 minutes. Then, these compounds were sequentially diluted 10-fold with Bravo, with a total of 10 concentrations, for testing. 750 nL of each compound was transferred to a 384-well compound plate with ECHO. Then, the compound plate was added with 30 μL of detection buffer.

d) Test steps:

The 384-well cell culture plate was taken out from the incubator, the culture medium was discarded, and 20 μL of test buffer and 20 μL of 2X Fluo-4 DirectTM No-Wash loading buffer were added with a pipette. The cell culture plate was incubated in a 5% $CO_2$ incubator at 37°C for 50 minutes and incubated at room temperature for 10 minutes. The cell culture plate, the compound plate and a tip box were placed into FLIPR, FLIPRTETRA test program was operated, 10 μL of reference compound and 10 μL of test compound were aspirated from the compound plate to the cell culture plate, fluorescence signals were read, and then data were processed. The data were analyzed by using Prism.

Specific test results are shown in FIG. 3. As shown in the figure, compound TRE exhibited strong agonist activity on the target IP receptor in a concentration-dependent manner, while prodrug compounds Ia-1, Ia-2 and Ia-3 provided in the present invention showed no agonist activity on the acting target IP receptor at any of the tested concentrations, demonstrating the safety of the compounds of the present invention as prodrug compounds.

Experimental Example 4. Experiment for in-vitro affinity of treprostinil derivative on EP2 receptor

This experiment was carried out in 96-well plates to evaluate the binding affinity of the compounds of the present invention (Ia-1, Ia-2 and Ia-3) to the prostaglandin EP2 receptor by testing their ability of competitive binding to the EP2 receptor with the radiolabeled ligand $^3$H-PGE2. Treprostinil was used as control. MicroBeta2 was used to detect the number of $^3$H captured, and Prism software was used to analyze data.

The EP2 film (Merck, Cat# HTS185M, batch number 2121326) was diluted with a buffer (50 mM Hepes, PH=7.4, 5 mM $MgCl_2$, 1 mM $CaCl_2$, 0.5% BSA) to a final concentration of 75 μg/mL. $^3$H-PGE2 was diluted to 15 nM. Test compound and control compound solutions with an initial concentration of 10 μM were prepared, and subjected to 10-fold continuous gradient dilution, with a total of 8 concentrations for testing. 5%DMSO was used as a solvent control group.

1 μL of each compound solution was added into a corresponding position in the 96-well test plate, and each well was added with 50 μL of the EP2 film (about 3.75 μg/well), and added with 50 μ L of $^3$H-PGE2. Then, the well plate was sealed, and incubated with shaking at 300 rpm at room temperature for 2 hours. After finishing the incubation, GF/C plates were filtered with Perkin Elmer Filtermate Harvester, and then each plate was washed with cold eluent for 4 times, and dried at 50°C for 1 hour. After drying, bottoms of wells of the filter plate were sealed with a PerkinElmer Unifilter-96 backing tape. 50 μL of Perkin Elmer Microscint O was added. The top of the filter plate was sealed with Perkin Elmer TopSeal-A sealing film. The number of $^3$H captured was detected by MicroBeta2.

Specific experimental results are shown in FIG. 4. The compound TRE competitively inhibited the binding of the ligand $^3$H-PGE2 to the EP2 receptor in a concentration-dependent manner, which indicated that, with the increase of concentration of TRE, the binding ability for the EP2 receptor was enhanced, while the prodrug compounds Ia-1, **Ia-2** and Ia-3 provided in the present invention showed no detectable binding to the EP2 receptor at any of the tested concentration, which proved the safety of the compounds of the present invention as the prodrug compounds.

Experimental Example 5. Study on efficacy of each compound after airway nebulized administration in U46619-induced rat acute pulmonary arterial pressure rise model

1. Experimental purpose:

U46619 was a stable analogue of thromboxane A2 (TXA2), which activated the downstream IP3 signal by binding to the thromboxane A2 receptor to promote vasoconstriction, platelet aggregation and the like. Influences of compound C16-TR and equimolar compounds Ia-1, Ia-2, Ia-3, R-11 and R-12 at different time points after airway nebulized administration on pulmonary hypertension of rats were studied by using the U46619-induced rat pulmonary arterial pressure rise mode, which were compared with that of equimolar TRE after airway nebulized administration.

2. Experimental materials

2.1 Main instruments

MP160-type 16-channel physiological recorder, rat aerosol pulmonary drug delivery device/intratracheal drug

delivery device, rat laryngoscope, animal anaesthesia machine, syringe, rat fixator, and the like.

2.2 Main reagents

dimethyl sulfoxide, PBS, methyl acetate, sodium chloride solution, 10% urethane, isoflurane and heparin saline. Modeling reagent: U46619 (9,11-dideoxy-11A,9A-methanoepoxy-prostaglandin F2A).

2.3 Experimental animals

SPF-grade male SD rats, 34 rats in total, about 250-400 g weight.

2.4 Preparation of test sample

Preparation of test sample: a required amount of compound TRE was taken and added into a sterile EP tube, and each tube was added with an appropriate amount of DMSO for dissolution to prepare a stock solution (100 mg/mL). Compounds C16-TR, Ia-1, Ia-2, Ia-3, R-11 and R-12 were added with appropriate amounts of DMSO and dissolved in the original bottles to prepare stock solutions (100 mg/mL), and then the stock solutions were transferred to sterile EP tubes. Each above stock solution was hermetically stored in the dark in a refrigerator at 4°C. When the stock solution was tested, an appropriate amount of stock solution was taken and added into a new sterile EP tube, and diluted with DMSO and 1×PBS to a target concentration (working solution) wherein the solvent was 3% DMSO/1×PBS.

Preparation of modeling agent: 900 μL of methyl acetate was added into an original bottle of U46619 and mixed evenly to prepare 1 mg/mL(1 μg/μL) stock solution, which was divided into 10 μL (1 μg/μL) per sterile EP tube and stored in a refrigerator at -20°C. On the day of modeling, the stock solution was taken out and balanced at room temperature and constant temperature, and each tube was added with an appropriate amount of 0.9% sodium chloride solution (sterile) to be diluted 20-fold, so as to prepare a working solution with a concentration of 50 ng/μL.

2.5 Animal grouping and administration

Airway nebulized administration dosages of compound TRE and the test compounds were equimolar quantities calculated based on TRE. The dosage of compound TRE group (n=3) was 13.3 μg/kg, the dosage of compound C16-TR group (n=5) was 20.9 μg/kg, the dosage of compound Ia-1 group (n=3) was 21.2 μg/kg, the dosage of compound Ia-2 group (n=6) was 22.1 μg/kg, the dosage of compound Ia-3 group (n=6) was 23.1 μg/kg, the dosage of compound R-11 group (n=3) was 21.9 μg/kg, and the dosage of compound R-12 group (n=3) was 21.7 μg/kg. There were also a model control group (n=1) and a solvent control group (n=3).

The SD rats were injected with U46619 (25 μg /kg) through the left jugular vein at different time points after airway nebulized administration, the peak value of RVSP rise after injection with U46619 was observed and recorded, and the inhibition percentage on the RVSP rise was calculated by comparing with the basic RVSP value. The rats in the model control group were administered with U46619 by intravenous injection immediately after successful catheterization and measurement of the basic RVSP. Intravenous injection time points of U46619 to the rats in other test compound groups were as shown in Table-2. RVSP of all animals were detected by right cardiac catheterization, and respiratory conditions of the animals were observed generally.

Table-2 Time points of administrating U46619 in various groups

| | 16 minutes | 1 hour | 3 hours | 6 hours | 16 hours | 24 hours | 48 hours |
|---|---|---|---|---|---|---|---|
| TRE | • | • | • | | | | |
| C16-TR | | • | • | • | • | • | • |
| Ia-1 | | • | | • | • | | |
| Ia-2 | | • | • | • | • | • | • |
| Ia-3 | | • | • | • | • | • | • |
| R-11 | | • | | • | • | | |
| R-12 | | • | | • | • | | |

2.6 Results

2.6.1 Results of general observation

Under the experimental conditions, the SD rats in all groups excluding the model control group were anesthetized with isoflurane, and then immediately administered by an airway nebulization, and no abnormal breathing was found after administration.

2.6.2 Results of right ventricular systolic pressure (RVSP)

All of the test compounds had inhibitory effects on the U46619-induced RVSP rise. In the reference Richard W. Chapman, et al. (2018 Pulmonary Pharmacology & Therapeutics), the effective critical value of the compound was set to be 30% inhibition percentage on the RVSP rise.The inhibition percentage on the U46619-induced RVSP rise of the experimental animals in each group at each time point was as shown in FIG. 5.

As shown in FIG. 5(a), RVSP of two control compounds R-11 and R-12 were recovered to the basic pressure or higher than the basic pressure at 6 hours after airway nebulized administration, which meant that the compounds basically had no antihypertensive effect. In FIG. 5(b), all of the compounds provided by the present invention and the control compound C16-TR had long-term antihypertensive effects. Antihypertensive effects of compound Ia-1 were better than those of C16-TR at 1 hour and 6 hours, but similar to C16-TR at 16 hours, both had no antihypertensive effect. Compound Ia-2 and compound Ia-3 had similar long-term effects, stronger antihypertensive effects than C16-TR and longer durations, and obvious antihypertensive effects could still be observed at 24 hours.

Experimental Example 6. Study on efficacies of compound C16-TR and test compounds after low-dose airway nebulized administration on U46619-induced rat acute pulmonary arterial pressure rise

1. Experimental purpose:

In this experiment, airway nebulized administration dosages of the compounds were about 1/7 of airway nebulized administration dosages in Experimental Example 5. By using a U46619-induced rat acute pulmonary arterial pressure rise model, antihypertensive effects of the compounds of the present invention after airway nebulized administration were further verified by reducing the administration dosages.

2. Animal grouping and administration:

A model construction method was the same as that described in Experimental Example 5. In this experiment, dosages of groups were as follows: the dosage of compound C16-TR group was 3 μg/kg, and the dosages of equimolar compounds Ia-1, Ia-2 and Ia-3 were 3.0 μg/kg, 3.2 μg/kg and 3.3 μg/kg respectively.

The SD rats were anesthetized and then administered by airway nebulization, and then the basic right ventricular systolic pressure (RVSP) was measured by right cardiac catheterization. The SD rats were intravenously injected with U46619 (25 μg /kg) at 1 hour after airway nebulized administration of each compound, a peak value of RVSP rise after injection with U46619 was observed and recorded, and the inhibition percentage on the RVSP rise was calculated by comparing with the basic RVSP value. The number of experimental animals in each group was 1~2.

3. Experimental results

Results of this experiment were as shown in Table-3 and FIG. 6. Even if the dosages were reduced by 7 times, the compounds of the present invention could still achieve significant antihypertension (which meant to inhibit the U46619-induced RVSP rise) at 1 hour after airway nebulized administration. This antihypertensive effect was even stronger than that of 13.3 μg/kg TRE at 16 minutes after airway nebulized administration in Experimental Example 5 (the inhibition percentage on the RVSP rise was about 39%), which was an exciting result. However, the control compound C16-TR had no antihypertensive effect at this moment. It was indicated that the compounds of the present invention could be converted into TRE more effectively, with faster onset speed and stronger antihypertensive effect.

Table-3 Percentage inhibitions of low-dose test compounds on RVSP rise at 1 hour after airway nebulized administration

| Group | Dosage | Inhibition percentage on RVSP rise (%) (mean) |
|---|---|---|
| C16-TR | 3 μg/kg | 5.49% |
| Compound Ia-1 | 3.04 μg/kg | 67.67% |

(continued)

| Group | Dosage | Inhibition percentage on RVSP rise (%) (mean) |
|---|---|---|
| Compound Ia-2 | 3.18 μg/kg | 65.78% |
| Compound Ia-3 | 3.31 μg/kg | 83.49% |

The above experimental results showed that the compounds provided by the present invention could be effectively converted into TRE to play a role in antihypertension after pulmonary administration. Compared with the control compound, the unique side chain structures of the compounds of the present invention endowed the compounds with stronger lung tissue affinities and unique local diffusion microdynamic characteristics, so that the compounds of the present invention could exert long-lasting drug effects as the prodrugs, and had excellent rapid onset effects at the same time. It was indicated that, after pulmonary administration, the compounds of the present invention could significantly reduce the dosages, reduce the adverse reactions due to systemic drug exposure, and reduce the risk of adverse reactions such as cough due to local high-concentration active ingredient exposure in lungs at the same time. The long-lasting and effective antihypertensive effects of the compounds were expected to reduce the administration frequency and provide a more effective, convenient and well-tolerated treatment choice for the patient with pulmonary hypertension, so that the compounds had far-reaching clinical values and advantages.

Although the present application has been described with reference to the specific embodiments, those skilled in the art shall understand that various changes and substitutions with equivalents can be made without departing from the true spirit and scope of the present application. In addition, various modifications can be made to adapt specific situations, materials, substances, methods and combinations of method steps to the objective spirit and scope of the present disclosure. All of such modifications are intended to be within the scope of the appended claims.

The patents, patent applications and journal articles cited in the present application are all incorporated by reference for all purposes.

## Claims

**1.** A compound of Formula I, an isomer or a pharmaceutically acceptable salt thereof,

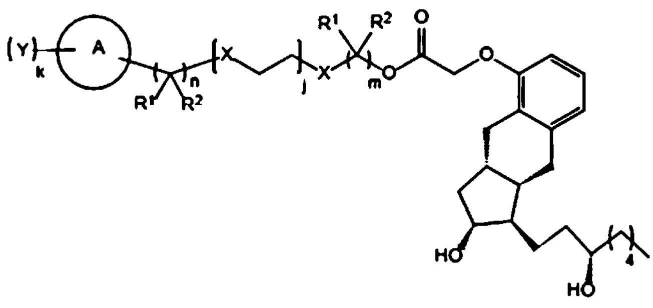

Formula I

wherein,

X is each independently selected from the group consisting of O and S;
A is a cyclic group, and the cyclic group is selected from the group consisting of phenyl and 5- to 10-membered heteroaryl, wherein the heteroatom in the 5- to 10-membered heteroaryl is selected from one or more of N, O and S;
$R^1$ and $R^2$ are each independently selected from the group consisting of H, $C_1$-$C_6$ alkyl, $C_2$-$C_6$ alkenyl, $C_2$-$C_6$ alkynyl, $C_1$-$C_6$ alkoxy, halogen atom, hydroxyl, amino, cyano, nitro and $-ONO_2$, or $R^1$ and $R^2$ form a cyclic group together with the carbon atom to which $R^1$ and $R^2$ are attached, wherein, the $C_1$-$C_6$ alkyl, $C_2$-$C_6$ alkenyl and $C_1$-$C_6$ alkoxy are optionally substituted by one or more substituents selected from the following groups: cycloalkyl, aryl, heteroaryl, $-NR^3R^4$, halogen atom, hydroxyl, cyano, mercapto, nitro or $-ONO_2$;
$R^3$ and $R^4$ are each independently selected from the group consisting of H, $C_1$-$C_6$ alkyl and $C_3$-$C_6$ cycloalkyl, or $R^3$ and $R^4$ form a 5-8 membered heterocyclic ring together with the nitrogen atom to which $R^3$ and $R^4$ are attached;
Y is each independently selected from the group consisting of $C_1$-$C_6$ alkyl, $C_2$-$C_6$ alkenyl, $C_2$-$C_6$ alkynyl, halogen, hydroxyl, carboxyl, cyano, mercapto, $C_1$-$C_6$ alkoxy, $C_1$-$C_6$ alkylamino and 3-8 membered cycloalkyl;
m is an integer from 4 to 16;

n is an integer from 0 to 8;
j is an integer from 0 to 2; and
k is an integer from 0 to 3.

**2.** The compound, the isomer or the pharmaceutically acceptable salt thereof according to claim 1, wherein, A is phenyl.

**3.** The compound, the isomer or the pharmaceutically acceptable salt thereof according to claim 1 or 2, wherein X is each independently O.

**4.** The compound, the isomer or the pharmaceutically acceptable salt thereof according to any one of claims 1 to 3, wherein $R^1$ and $R^2$ are each independently selected from H or $C_{1-6}$ alkyl, wherein the $C_{1-6}$ alkyl is optionally substituted by one or more substituents selected from the following groups: halogen atom, hydroxyl, cyano, amino, nitro, $-ONO_2$ and mercapto.

**5.** The compound, the isomer or the pharmaceutically acceptable salt thereof according to any one of claims 1 to 3, wherein Y is each independently selected from the group consisting of $C_1$-$C_6$ alkyl, halogen, cyano and $C_1$-$C_6$ alkoxy.

**6.** The compound, the isomer or the pharmaceutically acceptable salt thereof according to any one of claims 1 to 3, wherein Y is each independently chlorine.

**7.** The compound, the isomer or the pharmaceutically acceptable salt thereof according to any one of claims 1 to 3, wherein m is an integer from 6 to 10.

**8.** The compound, the isomer or the pharmaceutically acceptable salt thereof according to any one of claims 1 to 3, wherein n is an integer from 4 to 6.

**9.** The compound, the isomer or the pharmaceutically acceptable salt thereof according to any one of claims 1 to 3, wherein j is an integer from 0 to 1.

**10.** The compound, the isomer or the pharmaceutically acceptable salt thereof according to any one of claims 1 to 3, wherein k is an integer from 0 to 2.

**11.** A compound, an isomer or a pharmaceutically acceptable salt thereof, selected from a group of the following compounds:

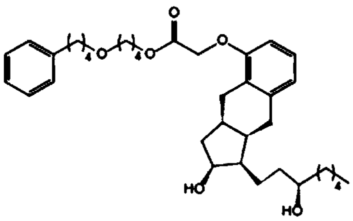 , 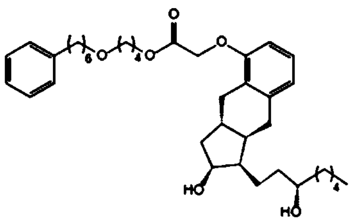 , 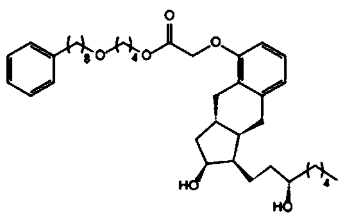 ,

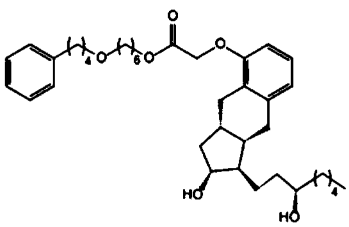 , 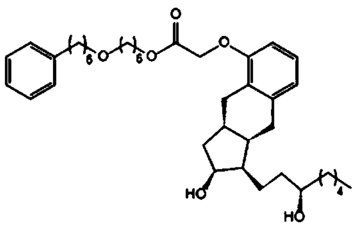 , 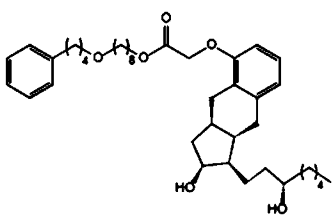 ,

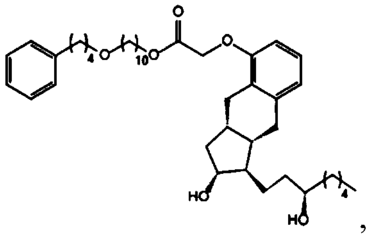 , 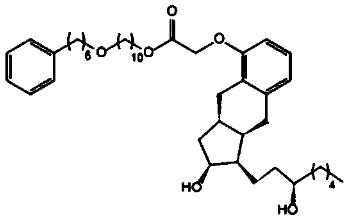 , 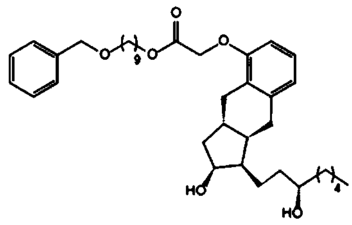 ,

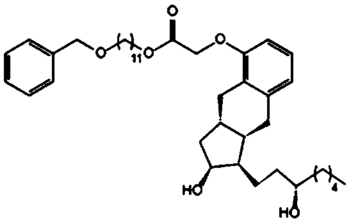 , 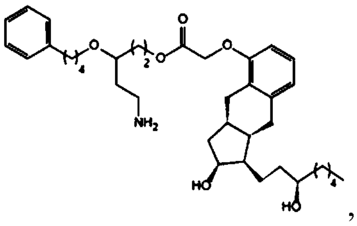 , 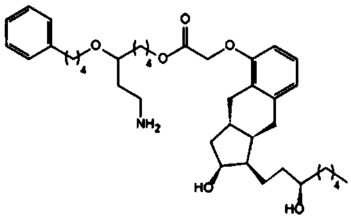 ,

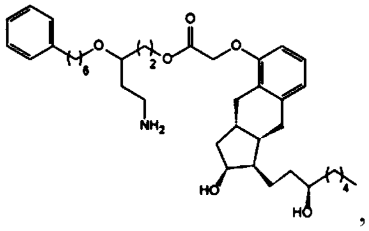 , 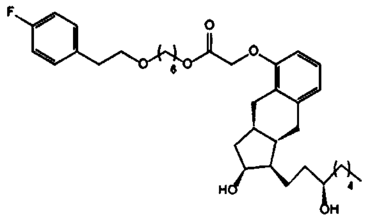 , 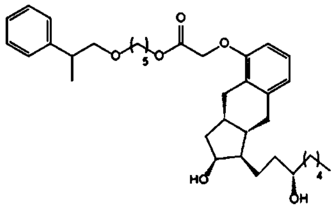 ,

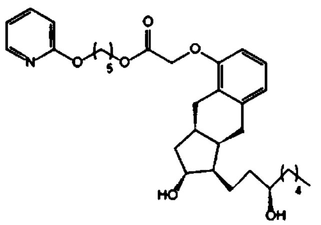 , 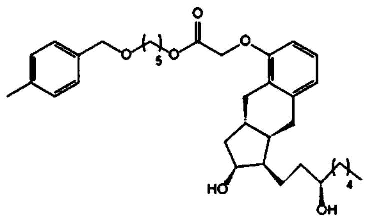 , 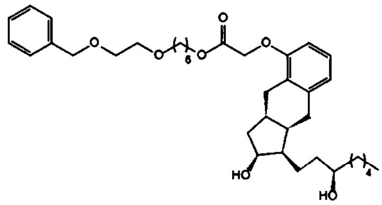 ,

and

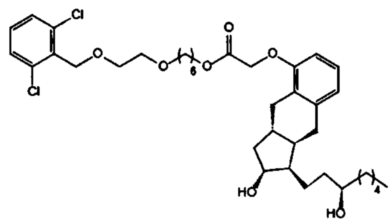 .

12. An isotope substitute of the compound or the pharmaceutically acceptable salt thereof according to any one of claims 1 to 11, wherein the isotope substitute is preferably a deuterium atom substitute.

13. A pharmaceutical composition, comprising the compound of Formula I, the isomer or the pharmaceutically acceptable salt thereof according to any one of claims 1 to 11, or the isotope substitute according to claim 12, and a pharmaceutically acceptable excipient.

14. A use of the compound, the isomer or the pharmaceutically acceptable salt thereof according to any one of claims 1 to 11, the isotope substitute according to claim 12, or the pharmaceutical composition according to claim 13 in the preparation of a medicament for treating pulmonary hypertension.

15. A use of the compound, the isomer or the pharmaceutically acceptable salt thereof according to any one of claims 1 to

11, the isotope substitute according to claim 12, or the pharmaceutical composition according to claim 13 in the preparation of a medicament for treating pulmonary fibrosis.

**16.** The use according to claim 14 or 15, comprising administering an effective dose of the compound, the isomer or the pharmaceutically acceptable salt thereof according to any one of claims 1 to 11, the isotope substitute according to claim 12, or the pharmaceutical composition according to claim 13 to a patient in need thereof.

**17.** The use according to claim 16, wherein the drug adopts pulmonary administration.

**18.** The use according to claim 17, wherein the pulmonary administration comprises administrating through a metered dose inhaler, a dry powder inhaler, a nebulizer and a soft mist inhaler.

**19.** The use according to claim 17, wherein the pulmonary administration comprises administrating through a metered dose inhaler.

**20.** The use according to claim 17, wherein the pulmonary administration comprises administrating through a dry powder inhaler.

**21.** The use according to claim 17, wherein the pulmonary administration comprises administrating through a nebulizer.

**22.** The use according to claim 17, wherein the pulmonary administration comprises administrating through a soft mist inhaler.

**23.** The use according to claim 14, wherein a patient suffering from the pulmonary hypertension is a patient with World Health Organization group I pulmonary hypertension.

**24.** The use according to claim 14, wherein a patient suffering from the pulmonary hypertension is a patient with World Health Organization group II pulmonary hypertension.

**25.** The use according to claim 14, wherein a patient suffering from the pulmonary hypertension is a patient with World Health Organization group III pulmonary hypertension.

**26.** The use according to claim 14, wherein a patient suffering from the pulmonary hypertension is a patient with World Health Organization group IV pulmonary hypertension.

**27.** The use according to claim 14, wherein a patient suffering from the pulmonary hypertension is a patient with World Health Organization group V pulmonary hypertension.

**28.** The use according to claim 14, wherein the pulmonary hypertension is selected from the group consisting of pulmonary Pulmonary arterial hypertension, PH due to left heart diseases, PH due to lung diseases and/or hypoxia, Chronic thromboembolic pulmonary hypertension, PH due to pulmonary arterial obstructive diseases, or PH associated with systemic or metabolic diseases.

**29.** The use according to claim 28, wherein the pulmonary arterial hypertension is selected from the group consisting of idiopathic pulmonary arterial hypertension, heritable pulmonary arterial hypertension, pulmonary arterial hypertension associated with congenital heart diseases, and pulmonary arterial hypertension associated with portal hypertension.

**30.** The use according to claim 28, wherein the pulmonary arterial hypertension is idiopathic pulmonary arterial hypertension.

**31.** The use according to claim 28, wherein the pulmonary arterial hypertension is heritable pulmonary arterial hypertension.

**32.** The use according to claim 28, wherein the pulmonary hypertension is PH due to left heart diseases.

**33.** The use according to claim 28, wherein the pulmonary hypertension is the PH due to lung diseases and/or hypoxia.

**34.** The use according to claim 33, wherein the PH due to lung diseases and/or hypoxia is pulmonary hypertension associated with interstitial lung diseases, pulmonary hypertension due to chronic obstructive pulmonary diseases, pulmonary hypertension due to combined pulmonary fibrosis and emphysema.

**35.** The use according to claim 28, wherein the pulmonary hypertension is Chronic thromboembolic pulmonary hypertension.

**36.** The use according to claim 28, wherein the pulmonary hypertension is the pulmonary hypertension associated with systemic or metabolic diseases.

**37.** The use according to claim 36, wherein the pulmonary hypertension associated with systemic or metabolic diseases is pulmonary hypertension associated with sarcoidosis.

**38.** The use according to claim 15, wherein the pulmonary fibrosis is selected from the group consisting of pulmonary interstitial fibrosis due to idiopathic interstitial pneumonias and pulmonary interstitial fibrosis due to connective tissue diseases.

**39.** The use according to claim 38, wherein the pulmonary interstitial fibrosis due to the idiopathic interstitial pneumonias is idiopathic fibrosis.

**40.** The use according to any one of claims 14, 15 and 17 to 22, wherein the effective dose of the compound of Formula I, the isomer or the pharmaceutically acceptable salt thereof is administered to the patient once daily.

**41.** The use according to any one of claims 14, 15 and 17 to 22, wherein the effective dose of the compound of Formula I, the isomer or the pharmaceutically acceptable salt thereof is administered to the patient once every two days.

**42.** The use according to any one of claims 14, 15 and 17 to 22, wherein the effective dose of the compound of Formula I, the isomer or the pharmaceutically acceptable salt thereof is administered to the patient twice daily.

**43.** A method for preparing the compound of Formula I according to any one of claims 1 to 11, comprising the following step of:

reacting a compound of Formula II with a compound of Formula III to obtain the compound of Formula I,

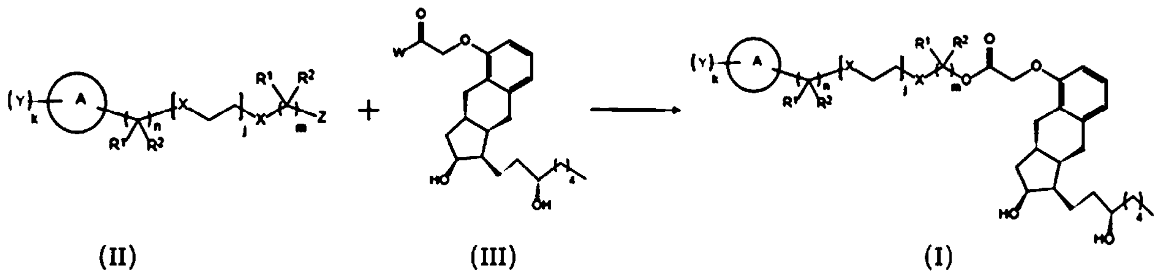

(II) (III) (I)

wherein, W is -OH, -Cl or $-OC(O)R_c$, and $R_c$ is $C_1$-$C_5$ alkyl;
wherein, Z is selected from -OH, -Cl, -Br and -I; and Y, A, $R^1$, $R^2$, X, n, j, m and k are as defined in claim 1.

**44.** The method according to claim 43, wherein, when W is -OH and Z is selected from -Cl, -Br and -I, the compound of Formula (II) is reacted with the compound of Formula (III) in an inert solvent in the presence of a base to obtain the compound of Formula I.

**45.** The method according to claim 44, wherein the base is an organic base or an inorganic base, wherein the organic base is selected from 1,8-diazabicyclo[5.4.0]undec-7-ene (DBU), N,N-diisopropylethylamine and/or diisopropylamine; and the inorganic base is selected from alkaline earth metal carbonate or hydroxide; the inert solvent is selected from ethyl acetate, N,N'-dimethylformamide, tetrahydrofuran, benzene, toluene, dioxane, dichloromethane, acetonitrile and/or polyhalogenated aliphatic hydrocarbon; the reaction is carried out at a temperature ranging from -20°C to 70°C, preferably 45°C to 65°C, for 1~8 hours, preferably 2~5 hours; and when Z is -Cl or -Br, the reaction is carried out in the presence of the alkaline earth metal carbonate, such as potassium carbonate.

**46.** The method according to claim 43, wherein, when W is -OH and Z is -OH, the compound of Formula (II) is reacted with the compound of Formula (III) in an inert solvent in the presence of a dehydrating agent and a catalyst to obtain the compound of Formula I.

**47.** The method according to claim 46, wherein the dehydrating agent is selected from dicyclohexylcarbodiimide or N'-(3-dimethylaminopropyl)-N-ethylcarbodiimide hydrochloride; the catalyst is selected from N,N-dimethylaminopyridine, sulfuric acid, sulfonic acid, hydrofluoric acid, phosphoric acid, toluenesulfonic acid, polystyrene sulfonate, heteropoly acid, zeolite, metal oxide, graphene oxide or a combination thereof; the inert solvent is selected from N,N-dimethylformamide, tetrahydrofuran, benzene, toluene, dioxane, dichloromethane, acetonitrile and/or polyhalogenated aliphatic hydrocarbon; and the reaction is carried out at a temperature ranging from -20°C to 70°C for 30 minutes to 48 hours.

**48.** The method according to claim 43, wherein, when W is - Cl and Z is -OH, the compound of Formula (II) is reacted with the compound of Formula (III) in an inert solvent in the presence of an organic base to obtain the compound of Formula I.

**49.** The method according to claim 48, wherein the organic base is selected from N,N-dimethylaminopyridine, triethylamine and/or pyridine; the inert solvent is selected from DMF, tetrahydrofuran, benzene, toluene, dioxane and/or halogenated aliphatic hydrocarbon; and the reaction is carried out at a temperature ranging from -20°C to 70°C for 30 minutes to 24 hours.

**50.** The method according to claim 43, wherein, when W is $-OC(O)R_c$ and Z is -OH, the compound of Formula (II) reacts with the compound of Formula (III) in an inert solvent in the presence of a catalyst to obtain the compound of Formula I.

**51.** The method according to claim 50, wherein the catalyst is selected from N,N-dimethylaminopyridine, an alkali metal compound catalyst, a Lewis acid catalyst, an alkaline earth metal alkoxide catalyst and/or an organotitanium catalyst; the alkali metal compound catalyst is selected from NaOH, KOH, $Li_2CO_3$, $Na_2CO_3$, $NaHCO_3$, $K_2CO_3$, $Na_2WO_4$ or a combination thereof; the Lewis acid catalyst is selected from $AlCl_3$, $ZnCl_2$, $SnCl_4$, $TiCl_4$, $FeCl_3$, $MgCl_2$, $Pb(OAc)_2$, $Zn(OAc)_2$, $Al_2O(OAc)_4$ or a combination thereof; the alkaline earth metal alkoxide catalyst is selected from sodium methoxide, sodium ethoxide, magnesium methoxide or a combination thereof; the organotitanium catalyst is selected from butyl titanate $Ti(OC_4H_9)_4$, titanocene dichloride ($Cp_2TiCl_2$) or a combination thereof; the inert solvent is selected from N,N-dimethylformamide, tetrahydrofuran, benzene, toluene, dioxane, dichloromethane, acetonitrile and/or polyhalogenated aliphatic hydrocarbon; and the reaction is carried out at a temperature ranging from -20°C to 70°C for 30 minutes to 24 hours.

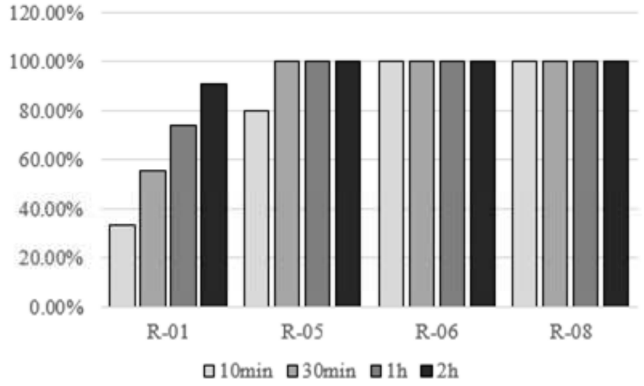


FIG. 1(a)

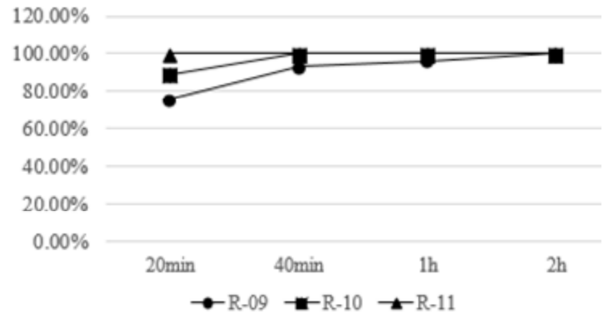


FIG. 1(b)

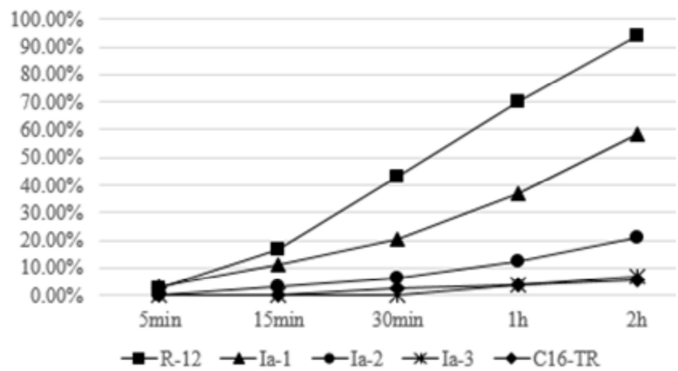


FIG. 1(c)

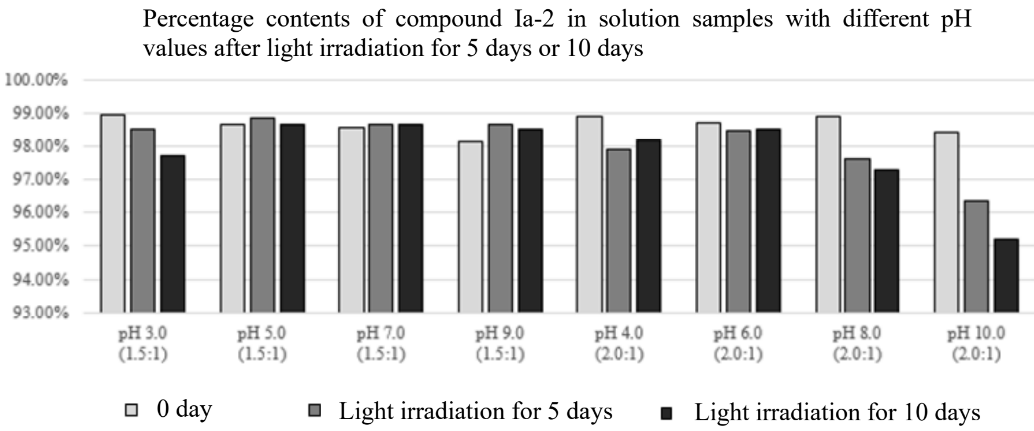

Percentage contents of compound Ia-2 in solution samples with different pH values after light irradiation for 5 days or 10 days
100.00%
99.00%
98.00%
97.00%
96.00%
95.00%
94.00%
93.00%
pH 3.0 (1.5:1)
pH 5.0 (1.5:1)
pH 7.0 (1.5:1)
pH 9.0 (1.5:1)
pH 4.0 (2.0:1)
pH 6.0 (2.0:1)
pH 8.0 (2.0:1)
pH 10.0 (2.0:1)
0 day
Light irradiation for 5 days
Light irradiation for 10 days


FIG. 2(a)

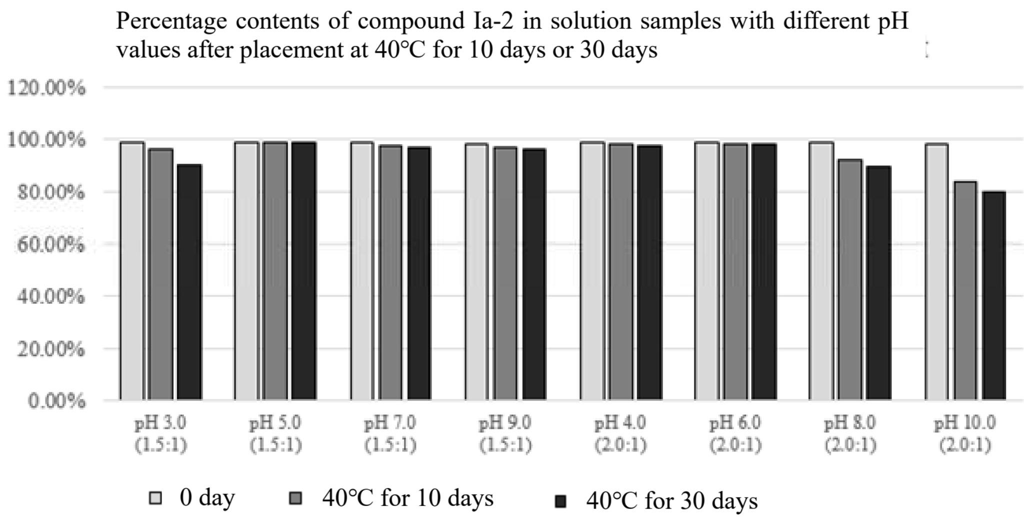

Percentage contents of compound Ia-2 in solution samples with different pH values after placement at 40°C for 10 days or 30 days
120.00%
100.00%
80.00%
60.00%
40.00%
20.00%
0.00%
pH 3.0 (1.5:1)
pH 5.0 (1.5:1)
pH 7.0 (1.5:1)
pH 9.0 (1.5:1)
pH 4.0 (2.0:1)
pH 6.0 (2.0:1)
pH 8.0 (2.0:1)
pH 10.0 (2.0:1)
0 day
40°C for 10 days
40°C for 30 days


FIG. 2(b)

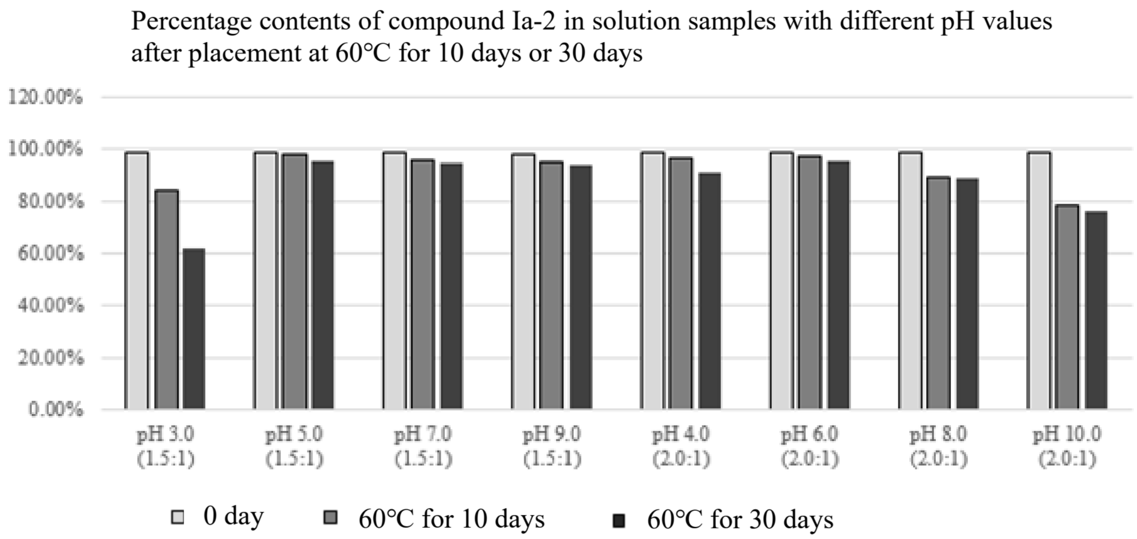

Percentage contents of compound Ia-2 in solution samples with different pH values after placement at 60°C for 10 days or 30 days
120.00%
100.00%
80.00%
60.00%
40.00%
20.00%
0.00%
pH 3.0 (1.5:1)
pH 5.0 (1.5:1)
pH 7.0 (1.5:1)
pH 9.0 (1.5:1)
pH 4.0 (2.0:1)
pH 6.0 (2.0:1)
pH 8.0 (2.0:1)
pH 10.0 (2.0:1)
0 day
60°C for 10 days
60°C for 30 days


FIG. 2(c)

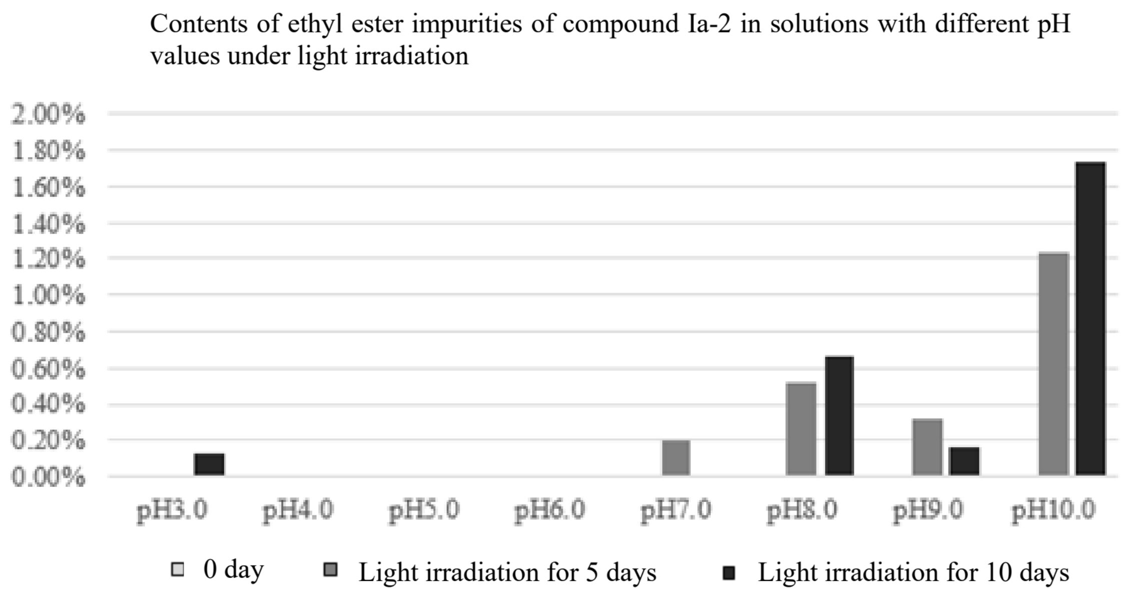

Contents of ethyl ester impurities of compound Ia-2 in solutions with different pH values under light irradiation
2.00%
1.80%
1.60%
1.40%
1.20%
1.00%
0.80%
0.60%
0.40%
0.20%
0.00%
pH3.0
pH4.0
pH5.0
pH6.0
pH7.0
pH8.0
pH9.0
pH10.0
0 day
Light irradiation for 5 days
Light irradiation for 10 days


FIG. 2(d)

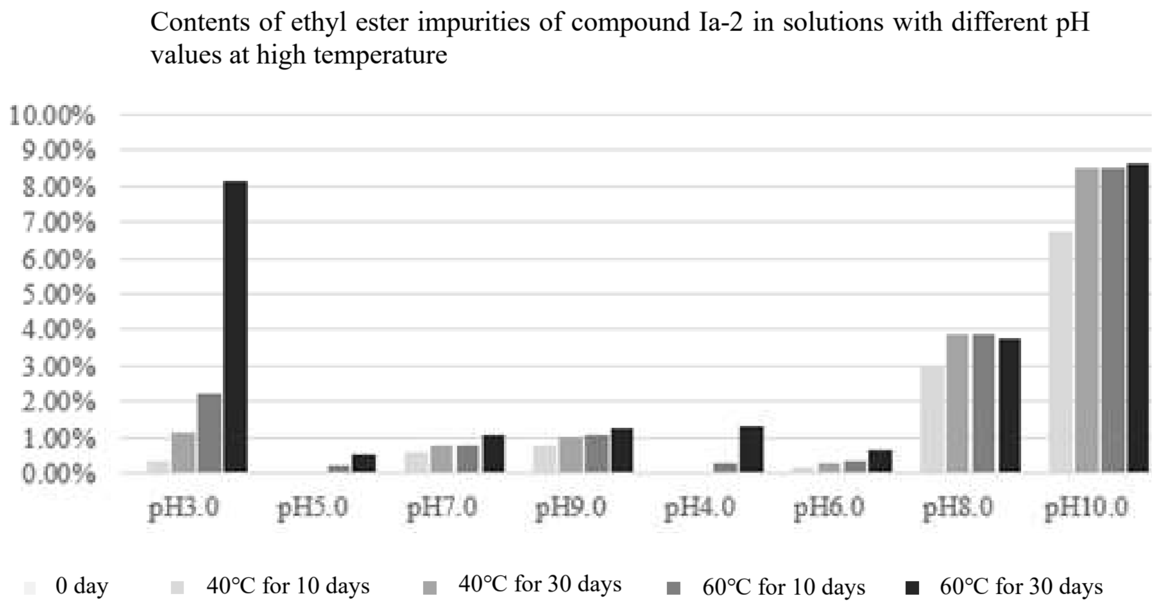


FIG. 2(e)

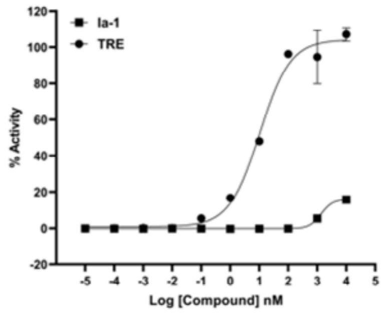


FIG. 3(a)

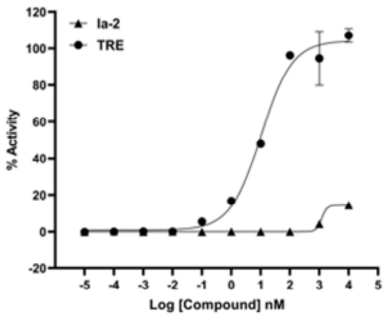


FIG. 3(b)

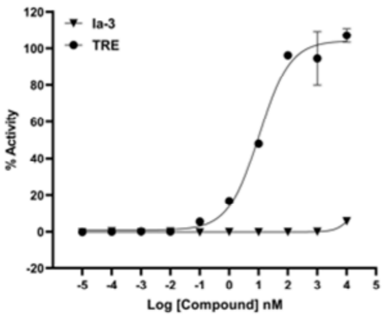


FIG. 3(c)

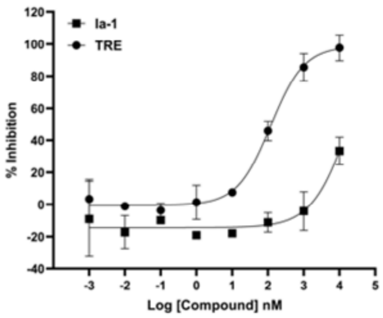


FIG. 4(a)

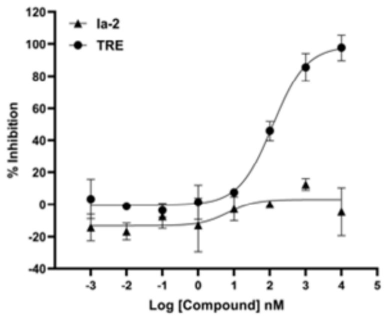


FIG. 4(b)

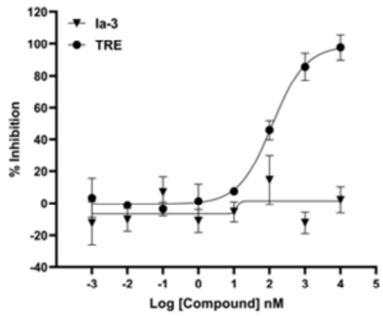


FIG. 4(c)

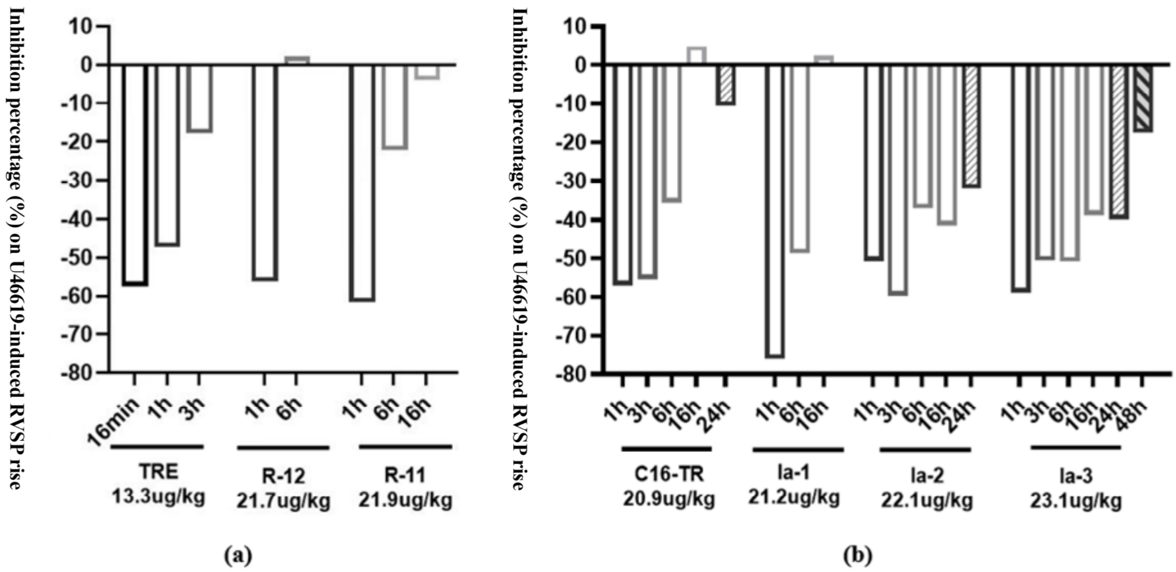
Inhibition percentage (%) on U46619-induced RVSP rise
10
0
-10
-20
-30
-40
-50
-60
-70
-80
16min
1h
3h
1h
6h
1h
6h
16h
TRE
13.3ug/kg
R-12
21.7ug/kg
R-11
21.9ug/kg
(a)
Inhibition percentage (%) on U46619-induced RVSP rise
1h
3h
6h
16h
24h
1h
6h
16h
1h
3h
6h
16h
24h
1h
3h
6h
16h
24h
48h
C16-TR
20.9ug/kg
Ia-1
21.2ug/kg
Ia-2
22.1ug/kg
Ia-3
23.1ug/kg
(b)

FIG. 5

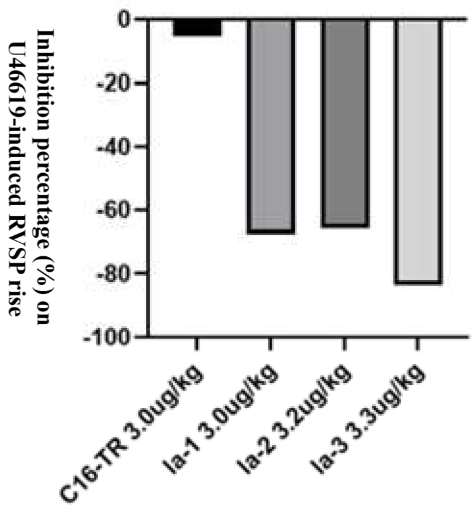
Inhibition percentage (%) on U46619-induced RVSP rise
0
-20
-40
-60
-80
-100
C16-TR 3.0ug/kg
Ia-1 3.0ug/kg
Ia-2 3.2ug/kg
Ia-3 3.3ug/kg

FIG. 6

## INTERNATIONAL SEARCH REPORT

International application No.
**PCT/CN2024/100071**

**A. CLASSIFICATION OF SUBJECT MATTER**

C07C 405/00(2006.01)i； A61K 31/335(2006.01)i； A61K 31/222(2006.01)i； A61P 9/00(2006.01)i

According to International Patent Classification (IPC) or to both national classification and IPC

**B. FIELDS SEARCHED**

Minimum documentation searched (classification system followed by classification symbols)

C07C, A61K, A91P

Documentation searched other than minimum documentation to the extent that such documents are included in the fields searched

Electronic data base consulted during the international search (name of data base and, where practicable, search terms used)

CNTXT, ENTXTC, DWPI, CNKI, STN (CAPLUS, REGISTRY, MARPAT): 肺动脉高压, 肺高压, 曲前列环素, 衍生物, 前药, PULMONARY, HYPERTENSION, TREPROSTINIL, PRODRUG, ANALOG, DERIVATIVE, structural formula search

**C. DOCUMENTS CONSIDERED TO BE RELEVANT**

| Category* | Citation of document, with indication, where appropriate, of the relevant passages | Relevant to claim No. |
|---|---|---|
| A | CN 105848479 A (INSMED INC.) 10 August 2016 (2016-08-10)<br>entire document | 1-51 |
| A | CN 108349926 A (CORSAIR PHARMACEUTICALS INC.) 31 July 2018 (2018-07-31)<br>entire document | 1-51 |
| A | CN 108947843 A (INSMED INC.) 07 December 2018 (2018-12-07)<br>entire document | 1-51 |
| A | CN 115448839 A (GUANGZHOU KAISHI MEDICINE CO., LTD. et al.) 09 December 2022 (2022-12-09)<br>entire document | 1-51 |
| A | CN 115894240 A (GUANGZHOU KAISHI MEDICINE CO., LTD. et al.) 04 April 2023 (2023-04-04)<br>entire document | 1-51 |
| A | US 9394227 B1 (CORSAIR PHARMACEUTICALS INC.) 19 July 2016 (2016-07-19)<br>entire document | 1-51 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **23 September 2024** | **25 September 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/100071**

**C. DOCUMENTS CONSIDERED TO BE RELEVANT**

| Category* | Citation of document, with indication, where appropriate, of the relevant passages | Relevant to claim No. |
|---|---|---|
| A | WO 2022194195 A1 (JIANGSU HENGRUI PHARMACEUTICALS CO., LTD.) 22 September 2022 (2022-09-22) entire document | 1-51 |

Form PCT/ISA/210 (second sheet) (July 2022)

**INTERNATIONAL SEARCH REPORT**
**Information on patent family members**

International application No.
**PCT/CN2024/100071**

| Patent document cited in search report | Publication date (day/month/year) | Patent family member(s) | Publication date (day/month/year) |
|---|---|---|---|
| CN 105848479 A | 10 August 2016 | KR 20210110401 A | 07 September 2021 |
| | | KR 102427785 B1 | 29 July 2022 |
| | | US 2016256425 A1 | 08 September 2016 |
| | | US 10010518 B2 | 03 July 2018 |
| | | PT 3060041 T | 12 March 2021 |
| | | JP 2020117552 A | 06 August 2020 |
| | | JP 6967111 B2 | 17 November 2021 |
| | | NZ 719297 A | 26 March 2021 |
| | | JP 2021185198 A | 09 December 2021 |
| | | JP 7190011 B2 | 14 December 2022 |
| | | JP 2023009145 A | 19 January 2023 |
| | | JP 7430766 B2 | 13 February 2024 |
| | | KR 20160074668 A | 28 June 2016 |
| | | KR 102296259 B1 | 30 August 2021 |
| | | MX 2020004912 A | 21 May 2021 |
| | | MX 2016005293 A | 12 August 2016 |
| | | ES 2860975 T3 | 05 October 2021 |
| | | US 2024109834 A1 | 04 April 2024 |
| | | US 2015175529 A1 | 25 June 2015 |
| | | US 9255064 B2 | 09 February 2016 |
| | | AU 2014339866 B2 | 15 March 2018 |
| | | AU 2014339866 C1 | 20 September 2018 |
| | | BR 112016009207 A8 | 24 March 2020 |
| | | BR 112016009207 B1 | 22 November 2022 |
| | | EP 3808731 A1 | 21 April 2021 |
| | | EP 3060041 A2 | 31 August 2016 |
| | | EP 3060041 A4 | 21 June 2017 |
| | | EP 3060041 B1 | 16 December 2020 |
| | | JP 2024009165 A | 19 January 2024 |
| | | WO 2015061720 A2 | 30 April 2015 |
| | | WO 2015061720 A3 | 11 June 2015 |
| | | SI 3060041 T1 | 30 April 2021 |
| | | DK 3060041 T3 | 22 March 2021 |
| | | AU 2019240559 A1 | 17 October 2019 |
| | | AU 2019240559 B2 | 09 April 2020 |
| | | AU 2018204198 A1 | 12 July 2018 |
| | | AU 2018204198 B2 | 25 July 2019 |
| | | US 2015148414 A1 | 28 May 2015 |
| | | US 9469600 B2 | 18 October 2016 |
| | | US 2020079724 A1 | 12 March 2020 |
| | | US 10995055 B2 | 04 May 2021 |
| | | US 2019248731 A1 | 15 August 2019 |
| | | US 10526274 B2 | 07 January 2020 |
| | | US 2017049739 A1 | 23 February 2017 |
| | | CY 1123944 T1 | 27 May 2022 |
| | | PL 3060041 T3 | 02 August 2021 |
| | | IL 245184 A0 | 30 June 2016 |
| | | IL 245184 B | 27 February 2020 |
| | | EA 201690623 A1 | 30 November 2016 |
| | | EA 031604 B1 | 31 January 2019 |

Form PCT/ISA/210 (patent family annex) (July 2022)

**INTERNATIONAL SEARCH REPORT**
**Information on patent family members**

International application No.
**PCT/CN2024/100071**

| Patent document cited in search report | Publication date (day/month/year) | Patent family member(s) | Publication date (day/month/year) |
|---|---|---|---|
| | | JP 2019056007 A | 11 April 2019 |
| | | JP 6722268 B2 | 15 July 2020 |
| | | JP 2016540734 A | 28 December 2016 |
| | | JP 6491203 B2 | 27 March 2019 |
| | | US 2021276940 A1 | 09 September 2021 |
| | | US 11795135 B2 | 24 October 2023 |
| | | CA 2927788 A1 | 30 April 2015 |
| | | CA 2927788 C | 06 September 2022 |
| | | HRP 20210351 T1 | 30 April 2021 |
| | | LT 3060041 T | 25 March 2021 |
| CN 108349926 A | 31 July 2018 | IL 289276 A | 01 February 2022 |
| | | IL 289276 B1 | 01 September 2023 |
| | | IL 289276 B2 | 01 January 2024 |
| | | JP 2018524304 A | 30 August 2018 |
| | | JP 7013245 B2 | 31 January 2022 |
| | | EP 3456715 A1 | 20 March 2019 |
| | | EP 3456715 B1 | 16 December 2020 |
| | | ES 2859749 T3 | 04 October 2021 |
| | | US 2018170855 A1 | 21 June 2018 |
| | | US 10246403 B2 | 02 April 2019 |
| | | US 2020071257 A1 | 05 March 2020 |
| | | US 11034645 B2 | 15 June 2021 |
| | | WO 2016205202 A1 | 22 December 2016 |
| | | IL 305157 A | 01 October 2023 |
| | | IL 305157 B1 | 01 July 2024 |
| | | JP 2024019523 A | 09 February 2024 |
| | | US 2018305293 A1 | 25 October 2018 |
| | | US 10464877 B2 | 05 November 2019 |
| | | EP 3310769 A1 | 25 April 2018 |
| | | EP 3310769 B1 | 18 August 2021 |
| | | KR 20180027517 A | 14 March 2018 |
| | | JP 2022003057 A | 11 January 2022 |
| | | ES 2897721 T3 | 02 March 2022 |
| | | US 2024124385 A1 | 18 April 2024 |
| | | US 2019241499 A1 | 08 August 2019 |
| | | US 10703706 B2 | 07 July 2020 |
| | | IL 256260 A | 28 February 2018 |
| | | IL 256260 B | 01 January 2022 |
| | | CA 2989317 A1 | 22 December 2016 |
| | | HK 1258913 A1 | 22 November 2019 |
| | | US 2021355072 A1 | 18 November 2021 |
| | | US 11866402 B2 | 09 January 2024 |
| | | EP 3835291 A1 | 16 June 2021 |
| | | US 2020347006 A1 | 05 November 2020 |
| | | US 10988435 B2 | 27 April 2021 |
| | | US 2024116847 A1 | 11 April 2024 |
| | | US 2022055980 A1 | 24 February 2022 |
| | | US 11802105 B2 | 31 October 2023 |
| | | US 2017298001 A1 | 19 October 2017 |
| | | US 9957220 B2 | 01 May 2018 |

Form PCT/ISA/210 (patent family annex) (July 2022)

**INTERNATIONAL SEARCH REPORT**
**Information on patent family members**

International application No.
**PCT/CN2024/100071**

| Patent document cited in search report | Publication date (day/month/year) | Patent family member(s) | Publication date (day/month/year) |
|---|---|---|---|
| | | US 2016368855 A1 | 22 December 2016 |
| | | US 9643911 B2 | 09 May 2017 |
| | | IL 313291 A | 01 August 2024 |
| CN 108947843 A | 07 December 2018 | None | |
| CN 115448839 A | 09 December 2022 | None | |
| CN 115894240 A | 04 April 2023 | None | |
| US 9394227 B1 | 19 July 2016 | US 2018016222 A1 | 18 January 2018 |
| | | US 10053414 B2 | 21 August 2018 |
| | | US 2019010112 A1 | 10 January 2019 |
| | | US 10464878 B2 | 05 November 2019 |
| | | US 2016368854 A1 | 22 December 2016 |
| | | US 9701616 B2 | 11 July 2017 |
| | | US 2021009501 A1 | 14 January 2021 |
| | | US 11407707 B2 | 09 August 2022 |
| | | US 2022363626 A1 | 17 November 2022 |
| | | US 2020095186 A1 | 26 March 2020 |
| | | US 10759733 B2 | 01 September 2020 |
| WO 2022194195 A1 | 22 September 2022 | TW 202246206 A | 01 December 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 105848479 B


### Non-patent literature cited in the description


- **RICHARD W. CHAPMAN et al.** *Pulmonary Pharmacology & Therapeutics*, 2018